# EUROPÄISCHE PATENTSCHRIFT

(11) **EP 3 232 999 B1**
(45) Veröffentlichungstag und Bekanntmachung des Hinweises auf die Patenterteilung: **26.01.2022**
(21) Anmeldenummer: 15823331.2
(22) Anmeldetag: 18.12.2015
(51) Int. Cl.: A61F 7/08, B65D 39/08, A61F 7/10

(54) **WÄRMFLASCHENSICHERHEITSVERSCHLUSS**
SAFETY CLOSURE FOR A HOT-WATER BOTTLE
FERMETURE DE SÉCURITÉ POUR BOUILLOTTE

(30) Priorität: 20.12.2014 DE 102014119307; 10.02.2015 DE 102015101853; 26.08.2015 DE 102015114180; 02.09.2015 DE 102015114671; 08.10.2015 DE 102015117217
(43) Veröffentlichungstag der Anmeldung: 25.10.2017
(73) Patentinhaber: WFI Wärmflascheninnovation UG (Haftungsbeschränkt), 81241 München (DE)
(72) Erfinder: OHMER, Benjamin, 80333 München (DE); JUNIOR, Volker, 82166 Gräfelfing (DE); DIEZ, Laura, 87439 Kempten (DE); KRAUTTER, Anja, 72459 Albstadt (DE)
(74) Vertreter: Kehl, Ascherl, Liebhoff & Ettmayr Patentanwälte Partnerschaft mbB
(86) Internationale Anmeldenummer: PCT/EP2015/080681
(87) Internationale Veröffentlichungsnummer: WO 2016/097386

(56) Entgegenhaltungen:
- EP-A2- 0 960 608
- WO-A1-2004/026595
- AU-A1- 2004 200 830
- FR-A- 819 762
- GB-A- 1 227 892
- US-A- 1 737 605
- US-A- 2 439 907
- US-A- 3 216 632
- US-A- 5 114 032

## Beschreibung

Die vorliegende Erfindung bezieht sich gemäß Anspruch 1 auf einen mehrteiligen Wärmflaschensicherheitsverschluss,

Die ersten aus Kunststoff oder Gummi bestehenden Wärmflaschen gab es seit den 1920er Jahren (http://de.wikipedia.org/wiki/W%C3%A4rmflasche#Geschichte). Seitdem hat sich das Produkt Wärmflasche so sehr verbreitet, dass nahezu jeder Haushalt über ein Exemplar verfügt.

Die Verbreitung der Wärmflasche erklärt sich z.B. dadurch, dass sie für sehr viele Anwendungsfälle geeignet ist und daher für sehr viele Menschen eine Bereicherung darstellt. So werden Wärmflaschen z.B. bei Krankheit, Unwohlsein oder zum lokalen Temperieren von Körperteilen, insbesondere bei Verspannungen, verwendet. Weiterhin wirkt die von der Wärmflasche gespendete Wärme oft beruhigend.

Wärmflaschen werden dabei in der Regel mit sehr heißem bis kochenden Wasser befüllt obwohl nahezu jede Wärmflasche einen Vermerk gemäß dem Britisch Standard BS 1970:2012 aufweist, der darauf hinweist, dass kein kochendes Wasser eingefüllt werden soll. Heißes, insbesondere nahezu kochendes Wasser, kann beim Kontakt mit der menschlichen oder tierischen Haut starke Verbrühungen verursachen, die dauerhafte Schäden bewirken können. Im Falle eines ungewünschten Öffnens der Wärmflasche kann das darin vorgehaltene Wasser austreten und die menschliche oder tierische Haut somit verbrühen.

Wärmflaschen sind ein sehr ausgereiftes Massenprodukt, von dem jährlich viele Million verkauft werden, wodurch sich bereits erschließt, dass erhebliche Ressourcen für die Verbesserung von Wärmflaschen eingesetzt wurden und werden. Ferner wurde bereits eine sehr große Menge an Schutzrechten veröffentlich, wodurch die intensive Beschäftigung mit dem Objekt "Wärmflasche" wiederspiegelt wird. So offenbaren z.B. die Druckschriften GB230731A und EP0960608A2 Vorrichtungen zum leichteren Öffnen von verschlossenen Wärmflaschen. Es wird dabei eine weitere Vorrichtung über dem Wärmflaschenverschluss angeordnet. Die weitere Einrichtung stellt dabei ein Hebelelement dar, durch das eine Verlängerung des auf den Verschluss wirkenden Hebelarms ausgebildet wird, wobei durch die Hebelarmverlängerung mit dem zunehmenden Hebelarm die zum Öffnen der Wärmflasche erforderlichen Kräfte sinken. In der EP0960608A2 wird die Ansicht vertreten, dass die zum Öffnen eines Wärmflaschenverschlusses erforderliche Kraft durch das Abkühlen der Wärmflasche ansteigt. Dies bedeutet jedoch auch, dass eine erst kürzlich mit heißem Wasser befüllte und nur unwesentlich abgekühlte Wärmflasche sich leicht öffnen lässt. Grundsätzlich offenbaren die Druckschriften GB230731A und EP0960608A2 die Verwendung gewöhnlicher Verschlüsse. Da die verwendeten Verschlüsse Standardverschlüsse sind, kann ein Kinder oder eine demente Person durch Kraftaufwendung auf das am Verschluss angeordnete Element zur händischen Kontaktierung ungewollt die mit heißem Wasser gefüllte Wärmflasche öffnen. Ferner offenbaren die Druckschriften DE19900197C2, DE 1906634U, DE1842006U, DE1645057U, DE1638820U, DE611569A, DE552591A, DE552930A, DE487404A, GB157173, WO2012/136535A1, GB496452, GB105735, GB160712, GB225915, GB227345, GB245968, GB246764, GB304903, GB604341, GB385822, GB1094364 Verschlüsse für Wärmflaschen, durch welche die Sicherheit oder der Komfort bei der Verwendung einer Wärmflasche erhöht werden soll.

Dennoch konnte keiner der zuvor genannten Gegenstände eine Lösung bereitstellen, aufgrund derer Brandverletzungen infolge der Verwendung einer Wärmflasche reduziert werden. So kann der Homepage www.paulinchen.de - einer Initiative für brandverletzte Kinder - hinsichtlich der Verwendung von Wärmflaschen entnommen werden: "Füllen Sie nur Wasser unter 50°Celsius in

Wärmflaschen und verschließen Sie sie gut" (vgl. http://www.paulinchen.de/fileadmin/content/2012_Downloads/Gefahren_in_Kueche_und_Bad.pdf Entnommen am 18.12.2014). Bekanntermaßen ist als "gut Verschließen" stets ein Verschließen mit hoher Kraft zu verstehen. Spielende Kinder oder demente Personen können jedoch unbewusst oder durch Zufall solche Verschlüsse betätigen und dadurch den Verschluss herausdrehen, wodurch das heiße Wasser aus der Wärmflasche austreten kann. Aufgrund der großen Öffnungsdurchmesser von mehr als 16 mm, insbesondere von mehr als 18 mm, kann das in eine Wärmflasche eingefüllte Wasser sehr schnell aus der Wärmflasche austreten, wodurch sehr schnell erhebliche Körperoberflächenteile der die Wärmflasche nutzenden Person verletzt bzw. verbrüht werden können.

Ferner ist ein Trend zu immer größeren Elementen zur händischen Kontaktierung erkennbar. Die zunehmende Größe der Elemente zur händischen Kontaktierung unterstützt dabei ein kraftarmes wasserdichtes Verschließen der Wärmflasche und ein kraftarmes Öffnen der Wärmflasche. Die Elemente zur händischen Kontaktierung von Wärmflaschen weisen Breiten von mehr als 3 cm oder von mehr als 4 cm oder von mehr als 4,5 cm auf.

Da Wärmflaschen auch Kindern oder dementen Personen ausgehändigt werden, besteht somit die Gefahr, dass diese Personen die frisch befüllte Wärmflasche öffnen und sich am auslaufenden Wasser verbrühen. Insbesondere demenzkranke Personen können oft große Kräfte aufbringen oder erinnern sich an die Funktion des Wärmflaschenverschlusses und betätigen dann diesen.

Es ist somit Aufgabe der vorliegenden Erfindung einen Wärmflaschensicherheitsverschluss bereitzustellen, der das zuvor genannte Problem löst bzw. eine wirksame Alternative zu den bekannten Verschlüssen bereitstellt.

Die zuvor gestellte Aufgabe wird erfindungsgemäß durch einen mehrteiligen Wärmflaschensicherheitsverschluss, insbesondere zum Verhindern eines unbefugten Öffnens einer Wärmflasche durch Dritte, gemäß Anspruch 1 gelöst. Der Wärmflaschensicherheitsverschluss umfasst mindestens ein Verschlussteil mit einem Außengewinde zum Eindrehen in ein Innengewinde einer Wärmflasche, und mit einem ersten Kraftübertragungselement zum Übertragen von Momenten zum Einschrauben des Verschlussteils in ein Innengewinde einer Wärmflasche zum wasserdichten Verschließen der Wärmflasche und zum Herausschrauben des Verschlussteils aus einem Innengewinde einer Wärmflasche; und ein Handbetätigungselement mit einem zweiten Kraftübertragungselement zum Einleiten der zum Einschrauben und Herausschrauben des Verschlussteils in ein Innengewinde einer Wärmflasche erforderlichen Momente in den Verschlussteil, wobei das erste Kraftübertragungselement und das zweite Kraftübertragungselement derart korrespondierend oder negativ zueinander ausgebildet sind, dass der Verschlussteil und das Handbetätigungselement unter Bildung einer Wirkverbindung, insbesondere eines Kraftschlusses und/oder eines Formschlusses und/oder Feldschlusses, lösbar miteinander koppelbar sind.

Alternativ wird die erfindungsgemäße Lösung durch einen mehrteiligen Wärmflaschensicherheitsverschluss zum Verhindern eines unbefugten Öffnens der Wärmflasche durch Dritte beschrieben, wobei ein Verschlussteil zum wasserdichten Verschließen einer Wärmflasche ausgebildet ist und ein weiteres mit dem Verschlussteil lösbar koppbares Handbetätigungselement zum Einschrauben des Verschlussteils in die Wärmflasche ausgebildet ist, wobei ausschließlich das Handbetätigungselement einen Formschlussbereich, insbesondere einen Hebel, zum manuellen Einleiten der zum Öffnen und wasserdichten Verschließen des Wärmflaschenverschlusses erforderlichen Drehmomente aufweist.

Der erfindungsgemäße Wärmflaschensicherheitsverschluss hat den Vorteil, dass mit ihm verschlossene Wärmflaschen auch an Personengruppen ausgehändigt werden können, welche die Gefahren eines unkontrollierten Öffnens nicht absehen können, wie z.B. Kinder oder demenzkranke Personen. Durch die mehrteilige Ausgestaltung des erfindungsgemäßen Wärmflaschensicherheitsverschlusses kann das zum Öffnen und Schließen erforderliche Handbetätigungselement nämlich an einem Platz verwahrt werden, an den diese Personen nicht gelangen können. Ferner kann der Verschlussteil auch nicht durch "herumspielen" zufällig herausgedreht werden, da die erforderliche Kraftaufwendung - bei korrekter Verschließung - ohne ein zu dem ersten Kraftübertragungselement korrespondierenden Elements nicht geöffnet werden. Weitere bevorzugte Ausführungsformen sind Gegenstand der Unteransprüche und der nachfolgenden Beschreibung.

Bevorzugt ist das Verschlussteil zum wasserdichten Verschließen einer Wärmflasche bzw. zum Einschrauben in eine Wärmflasche mit einem Drehmoment von mindestens 1,3 Nm und bevorzugt von mindestens 1,4 Nm oder von mindestens 1,5 Nm oder von mindestens 1,6 Nm oder von mindestens 1,7 Nm oder von mindestens 1,8 Nm oder von mindestens 1,9 Nm oder von 2Nm +/- 0,1 Nm und bevorzugt mit maximal 2,5 Nm oder maximal 2,6 Nm oder maximal 2,7 Nm oder maximal 2,8 Nm oder maximal 2,9 Nm oder maximal 3 Nm oder maximal 3,5 Nm oder maximal 4 Nm zu beaufschlagen.

Gemäß der Erfindung ist das erste Kraftübertragungselement als innenliegendes Kraftübertragungselement ausgeführt und wird durch umgebende Wandungsanteile ausgebildet, wobei das zweite Kraftübertragungselement zum zumindest abschnittsweisen Eindringen in das innenliegende Kraftübertragungselement bzw. in einen Eindringbereich des Verschlussteils ausgebildet ist oder das erste Kraftübertragungselement ist als ebenes Kraftübertragungselement ausgebildet, insbesondere weist es Klett auf.

Gemäß einer weiteren bevorzugten Ausführungsform ist das erste Kraftübertragungselement als hervorstehendes Kraftübertagungselement ausgebildete, wobei das hervorstehende Kraftübertragungselement bevorzugt maximal 3 cm, insbesondere maximal 2,9 cm oder 2,8 cm oder 2,7 cm oder 2,6 cm oder 2,5 cm oder 2,4 cm oder 2,3 cm oder 2,2 cm oder 2,1 cm oder 2,0 cm oder 1,9 cm oder 1,8 cm oder 1,7 cm oder 1,6 cm oder 1,5 cm oder 1,4 cm oder 1,3 cm oder 1,2 cm oder 1,1 cm oder 1,0 cm oder 0,9 cm oder 0,8 cm oder 0,7 cm oder 0,6 cm oder 0,5 cm über das benachbarte Ende des Gewindes hervorsteht und/oderbevorzugt eine maximale Breite von 2,9 cm, insbesondere von 2,8 cm oder 2,7 cm oder 2,6 cm oder 2,5 cm oder 2,4 cm oder 2,3 cm oder 2,2 cm oder 2,1 cm oder 2,0 cm oder 1,9 cm oder 1,8 cm oder 1,7 cm oder 1,6 cm oder 1,5 cm oder 1,4 cm oder 1,3 cm oder 1,2 cm oder 1,1 cm oder 1,0 cm oder 0,9 cm oder 0,8 cm oder 0,7 cm aufweist.

Das erste Kraftübertragungselement ist besonders bevorzugt als hervorstehendes Kraftübertagungselement ausgebildete, wobei durch das hervorstehende Kraftübertagungsmittel ein Hebelarm zum Einleiten des Drehmoments zum Öffnen und Verschließen einer Wärmflasche ausgebildet wird, wobei der Hebelarm kürzer ist als ein durch das Handbetätigungselement ausgebildeter Hebelarm zum Einleiten des Drehmoments zum Öffnen und Verschließen einer Wärmflasche. Diese Ausführungsform ist vorteilhaft, da in jedem Fall eine Gestalt bereitgestellt wird, die ein manuelles Öffnen der Wärmflasche ohne das Handbetätigungselement nicht ermöglicht. Bevorzugt ist der Hebelarm des Handbetätigungselements gegenüber dem Hebelarm des Verschlussteils um ein Vielfaches, insbesondere um mindestens das Doppelte oder mindestens das Dreifache oder mindestens das Vierfache oder mindestens das Fünffache oder mindestens das Sechsfache oder mindestens das Siebenfache oder mindestens das Achtfache oder mindestens das Neunfache oder mindestens das Zehnfache oder mindestens das Elffache oder mindestens das Zwölffache oder mindestens das Fünfzehnfache oder mindestens das Zwanzigfache, größer. Besonders bevorzugt weist das hervorstehende Kraftübertragungselement einen oder mehrere Hebelarme auf, wobei die maximale Länge eines Hebelarms oder eines jeden Hebelarms 2cm, insbesondere 1,9 cm oder 1,8 cm oder 1,7 cm oder 1,6 cm oder 1,5 cm oder 1,4 cm oder 1,3 cm oder 1,2 cm oder 1,1 cm oder 1,0 cm oder 0,9 cm oder 0,8 cm oder 0,7 cm oder 0,5 cm oder 0,4 cm beträgt.

Gemäß einer weiteren bevorzugten Ausführungsform der vorliegenden Erfindung ist die Kopplung zwischen Handbetätigungselement und Verschlussteil zumindest dann lösbar oder bevorzugt nur dann lösbar, wenn der Verschlussteil zum Verschließen einer Wärmflasche in das Innengewinde der Wärmflasche hineingeschraubt ist. Das Handbetätigungselement und der Verschlussteil sind in einem entkoppelten Zustand frei bzw. beliebig zueinander bewegbar.

Gemäß der Erfindung weist der Verschlussteil zumindest abschnittsweise und bevorzugt vollständig umlaufend einen Kragen auf, wobei der Kragen als Anstoß zur Begrenzung der maximalen Einschraubtiefe und als Dichtungselement dient. Bevorzugt ist am Kragen eine Dichtung angeordnet oder ausgebildet. Bevorzugt ist das Dichtungselement als ein umlaufendes Dichtungselement ausbildet, wobei das umlaufende Dichtungselement bevorzugt als eine Erhebung mit einer gebogenen Kontaktoberfläche ausgebildet ist und besonders bevorzugt einstückiger Bestandteil des Kragens und somit des Verschlussteils ist

Gemäß einer weiteren bevorzugten Ausführungsform sind der Verschussteil und/oder das Handbetätigungselement Spritzgussteile, die bevorzugt aus einem Polymerwerkstoff bestehen, wobei der Polymerwerkstoff eine Temperaturfestigkeit von mindestens 100°C und bevorzugt von mindestens 120°C oder von mindestens 150°C (bevorzugt bis 200° oder bis 300°C oder bis 500°C oder bis 800°C) aufweist.

Gemäß einer weiteren bevorzugten Ausführungsform ist das Verschlussteil zum Verschließen von Wärmflaschenöffnungen ausgebildet, die einen Öffnungsdurchmesser von mindestens 16 mm oder von mindestens 17 mm oder von mindestens 18 mm oder von mindestens 19 mm oder von mindestens 20 mm (bevorzugt bis zu 30 mm oder bis zu 40mm oder bis zu 50 mm) aufweisen

Die Erfindung bezieht sich ferner auf ein Handbetätigungselement zur Verwendung in einem Wärmflaschensicherheitsverschluss gemäß Anspruch 1. Das Handbetätigungselement umfasst dabei bevorzugt mindestens ein Kraftübertragungselement zum Einleiten der zum Einschrauben und Herausschrauben des Verschlussteils in ein Innengewinde einer Wärmflasche erforderlichen Momente in den Verschlussteil.

Die Erfindung bezieht sich ferner auf ein Verschlussteil zur Verwendung in einem Wärmflaschensicherheitsverschluss gemäß Anspruch 1. Das Verschlussteil umfasst dabei bevorzugt mindestens ein Außengewinde zum Eindrehen in ein Innengewinde einer Wärmflasche, und ein Kraftübertragungselement zum Übertragen von Momenten zum Einschrauben des Verschlussteils in ein Innengewinde einer Wärmflasche zum wasserdichten Verschließen der Wärmflasche und zum Herausschrauben des Verschlussteils aus einem Innengewinde einer Wärmflasche.

Gemäß der Erfindung wird zumindest ein Teil eines Eindringbereichs des Verschlussteils, in den das zweite Kraftübertragungselement einführbar ist, durch eine zumindest abschnittsweise und bevorzugt mehrheitlich und besonders bevorzugt vollständig den Eindringbereich umschließende Wandung begrenzt. Bevorzugt weist dabei das zweite Kraftübertragungselement zumindest abschnittsweise eine außenliegende Wandung auf, die mit der umschließenden Wandung des Eindringbereichs koppelbar ist. Koppelbar bedeutet hierbei bevorzugt kontaktierbar, insbesondere zum Übertragen von Kräften kontaktierbar. Alternativ wird der Eindringbereich bevorzugt zusätzlich zur umschließenden Wandung ebenfalls durch mindestens eine Außenwandung von mindestens einer im durch die umschließende Wandung begrenzten Bereich ausgebildeten Erhebung, insbesondere einem zapfenartigen oder säulenartigen Element, begrenzt. Bevorzugt ist die Erhebung somit in einem Bereich angeordnet, der von der umschließenden Wandung zumindest teilweise und bevorzugt mehrheitlich und besonders bevorzugt vollständig umgeben bzw. umschlossen wird.

Gemäß einer weiteren bevorzugten Ausführungsform ist das Kraftübertragungselement als innenliegendes Kraftübertragungselement ausgeführt und wird bevorzugt durch umgebende Wandungsanteile vollständig oder teilweise ausgebildet, oder das Kraftübertragungselement ist als ebenes Kraftübertragungselement ausgebildet, insbesondere weist es bevorzugt Klett auf, oder das Kraftübertragungselement ist als hervorstehendes Kraftübertagungselement ausgebildet, wobei das hervorstehende Kraftübertragungselement maximal 3 cm, insbesondere maximal 2,9 cm oder 2,8 cm oder 2,7 cm oder 2,6 cm oder 2,5 cm oder 2,4 cm oder 2,3 cm oder 2,2 cm oder 2,1 cm oder 2,0 cm oder 1,9 cm oder 1,8 cm oder 1,7 cm oder 1,6 cm oder 1,5 cm oder 1,4 cm oder 1,3 cm oder 1,2 cm oder 1,1 cm oder 1,0 cm oder 0,9 cm oder 0,8 cm oder 0,7 cm oder 0,6 cm oder 0,5 cm über das benachbarte Ende des Gewindes hervorsteht und/oder ein maximale Breite von 2cm, insbesondere von 1,9 cm oder 1,8 cm oder 1,7 cm oder 1,6 cm oder 1,5 cm oder 1,4 cm oder 1,3 cm oder 1,2 cm oder 1,1 cm oder 1,0 cm oder 0,9 cm oder 0,8 cm oder 0,7 cm aufweist.

Gemäß einer weiteren bevorzugten Ausführungsform sind das erste Kraftübertragungselement und das zweite Kraftübertragungselement korrespondierend ausgebildet, wobei das erste Kraftübertragungselement und das zweite Kraftübertragungselement zum Erzeugen der Wirkverbindung derart miteinander in Kontakt bringbar sind, dass die korrespondierenden Anteile des ersten Kraftübertragungselements und des zweiten Kraftübertragungselements miteinander in Kontakt kommen, wobei die korrespondierenden Anteile zumindest abschnittsweise, insbesondere flächenmäßig mehrheitlich oder vollständig, gebogen ausgebildet sind. Diese Lösung ist besonders vorteilhaft, da durch abgerundete Flächen - ohne das korrespondierende Kraftübertragungselement - nur sehr schwer mit der Hand oder weiteren Hilfsmitteln der Verschlussanteil aus einem eine Wärmflasche verschließenden Zustand in einen eine Wärmflasche nicht verschließenden Zustand überführbar ist.

Gemäß einer weiteren bevorzugten Ausführungsform ist das hervorstehende bzw. zapfenartige, insbesondere in Längsrichtung des Verschlussteils über die Kragenoberfläche hinausragendes, Kraftübertragungselement des Verschlussteils derart gestaltet, dass die Einleitung eines Drehmoments von 2Nm +/- 0,1 Nm in das Verschlussteil zum wasserdichten Verschließen und zum Öffnen einer Wärmflasche eine Kraftbeaufschlagung des hervorstehenden Kraftübertragungselements mit einer Kraft von mehr als50 N, insbesondere mehr als 55 N oder von mehr als 60 N oder von mehr als 65 N oder von mehr als 60 N oder von mehr als 65 N oder von mehr als 70 N oder von mehr als 75 N oder von mehr als 80 N oder von mehr als 85 N oder von mehr als 90 N oder von mehr als 95 N oder von mehr als 100N oder von mehr als 105 N oder von mehr als 110N oder von mehr als 115 N oder von mehr als 120N oder von mehr als 125 N oder von mehr als 130 N oder von mehr als 140 N oder von mehr als 150 N oder von mehr als 160 N oder von mehr als 170 N oder von mehr als 180 N oder von mehr als 190 N oder von mehr als 200 N oder von mehr als 210 N oder von mehr als 220 N oder von mehr als 230 N oder von mehr als 240 N oder von mehr als 250 N oder von mehr als 260 N oder von mehr als 270 N oder von mehr als 280 N oder von mehr als 290 N oder von mehr als 300 N oder von mehr als 310 N oder von mehr als 320 N oder von mehr als 330 N oder von mehr als 340 N oder von mehr als 350 N, erfordert. Diese Lösung ist vorteilhaft, da, insbesondere zielgruppenabhängig, das Verschlusselement derart gestaltet werden kann, dass die von den einzelnen Personen der Zielgruppe aufbringbaren Kräfte geringer sind als die zum Öffnen einer mit dem erfindungsgemäßen Wärmflaschensicherheitsverschluss verschlossenen Wärmflasche. Ferner ist denkbar, dass die über das hervorstehende Kraftübertragungsmittel in den Verschlussteil einzubringende Kraft so groß ist, dass sie von mehr als 90%, insbesondere von mehr als 99 %, der Personen dieser Zielgruppe, insbesondere Kinder und demente Personen, nicht geöffnet werden kann.

Die Erfindung bezieht sich ferner auf einen Satz bestehend aus einem mehrteiligen Wärmflaschensicherheitsverschluss gemäß Anspruch 1 und einer Wärmflasche mit einer Einfüllöffnung und einem im Bereich der Einfüllöffnung angeordnetes Gewinde zum Koppeln mit dem Wärmflaschensicherheitsverschluss und bevorzugt mit einem um die Wärmflasche herum anordenbaren Bezug bevorzugt zum zumindest teilweisen Isolieren der Wärmflasche bzw. zum zumindest teilweise reduzieren des Wärmetransports von der Wärmflasche zum Nutzer.

Gemäß einer weiteren bevorzugten Ausführungsform der vorliegenden Erfindung wirken das Verschlussteil und das Handbetätigungselement derart, insbesondere klemmend, Zusammen, dass durch das Zusammenwirken des ersten Kraftübertragungselements und des zweiten Kraftübertragungselements das erste Kraftübertragungselement und das zweite Kraftübertragungselement in einem gekoppelten Zustand gehalten werden. Diese Ausführungsform ist vorteilhaft, da durch das Klemmen eine Verbindung zwischen dem Handbetätigungselement und dem Verschlussteil erzeugt werden kann, durch die das Verschlussteil am Handbetätigungselement verbleibt, wenn es aus dem Gewinde herausgeschraubt ist. Selbstverständlich ist das Verschlussteil weiterhin vom Handbetätigungselement abziehbar, dennoch lässt sich die Wärmflasche durch die Ausführungsform leichter öffnen und der Wärmflaschensicherheitsverschluss kann als eine gekoppelte Baugruppe verstaut werden. Bevorzugt ist der Eindringbereich des Verschlussteils zumindest abschnittsweise weniger breit ausgebildet als das in den Eindringbereich eindringende zweite Kraftübertragungselement des Handbetätigungselements, woraus beim Einbringen des zweiten Kraftübertragungselements in den Eindringbereich eine Presspassung bzw. Klemmung erzeugbar ist.

Gemäß einer weiteren bevorzugten Ausführungsform weist das zweite Kraftübertragungselement zumindest abschnittsweise eine außenliegende Wandung auf, die mit der umschließenden Wandung des Eindringbereichs koppelbar ist, wobei bevorzugt der Eindringbereich zusätzlich zur umschließenden Wandung ebenfalls durch mindestens eine Außenwandung von mindestens einer im durch die umschließende Wandung begrenzten Bereich ausgebildeten Erhebung, insbesondere einem zapfenartigen oder säulenartigen Element, begrenzt wird. Somit bildet der Eindringbereich bevorzugt eine grabenartige Gestalt aus. Zusätzlich oder alternativ ist denkbar, dass der Eindringbereich als mehrere voneinander beabstandete Teileindringbereiche ausgebildet sind, die z.B. eine sacklochartige Gestalt aufweisen können. Ferner ist denkbar, dass der Eindringbereich abschnittsweise grabenartig und abschnittsweise durch einen Teileindringbereich oder mehrere Teileindringbereich ausgebildet wird. Durch die Wandungen, durch welche der Eindringbereich und/oder die Teileindringbereiche begrenzt werden, werden bevorzugt Teilkraftübertragungselement des Verschlussteils ausgebildet.

Die vorliegende Erfindung bezieht sich ferner auf ein Los. Das Los umfasst dabei bevorzugt mindestens eine Verpackung, insbesondere zumindest teilweise aus einem Polymermaterial bestehend, die einen Aufnahmebereich begrenzt, und ein von der Verpackung in dem Aufnahmebereich zumindest flüssigkeitsdicht eingeschlossener Satz gemäß Anspruch 3, wobei der gesamte von der Verpackung begrenzte Aufnahmebereich keimreduziert, insbesondere steril, ist. Im Rahmen der vorliegenden Erfindung ist der Begriff "steril" bevorzugt gemäß den Standards EN 556-1 und EN 552 zu verstehen, wobei die Anwendung bevorzugt auf Laborprodukte gerichtet ist und besonders bevorzugt auf Medizinprodukte gerichtet ist. Die Standards EN 556-1 und EN552 werden durch Bezugnahme vollumfänglich zum Gegenstand der vorliegenden Schrift erklärt. Als keimreduziert wird hierbei bevorzugt jeder Zustand verstanden, der durch eine Behandlung erreicht wird, die eine Reduzierung und/oder Inaktivierung von Biomasse, insbesondere wirksame DNA oder wirksame DNA-Fragmente oder Pathogene und/oder Mikroorganismen, insbesondere biologisch wirksame Bakterien und/oder biologisch wirksame Viren und/oder biologisch wirksame Sporen, bewirkt. Bevorzugt beschreibt der Begriff keimreduziert somit einen Zustand, der dem Sicherheitsgrad SAL 2 oder mindestens dem Sicherheitsgrad SAL 2, insbesondere SAL 3 oder mindestens SAL3 oder SAL 4 oder mindestens SAL4 oder SAL 5, entspricht. SAL steht hierbei für den in einheitlich verwendeten Begriff Sterility Assurance Level. Bevorzugt beschreibt der Begriff "steril" somit im Rahmen der vorliegenden Erfindung einen Zustand, der dem Sicherheitsgrad SAL 6 oder höher entspricht.

Gemäß einer bevorzugten Ausführungsform ist das Los, insbesondere der Aufnahmebereich mit den darin angeordneten Objekten, durch mindestens einem von einer

Strahlungsquelle, insbesondere einem Elektronenbeschleuniger, erzeugten Strahl, insbesondere Elektronenstrahl oder Gammastrahl oder UV-Lichtstrahl, zur zumindest Keimreduzierung, insbesondere Sterilisation, nach der Einbringung des Objekts und nach der Versiegelung der Verpackung beaufschlagt worden. Bevorzugt ist die Verpackung nicht zerstörungsfrei zu öffnen.

Zusätzlich oder alternativ ist hierbei denkbar, dass eine Keimreduzierung, insbesondere Sterilisation, mittels einer Gasbeaufschlagung der Verpackung und des darin eingebrachten Objekts bzw. der darin eingebrachten bzw. einzubringenden Objekte erfolgt bzw. erfolgte. Bevorzugt handelt es sich bei der Gasbeaufschlagung um ein Funktionsgasbeaufschlagung, insbesondere eine Ethylenoxid/EtO Beaufschlagung. Besonders bevorzugt ist die Verpackung bei der Gasbeaufschlagung bereits mit dem Objekt oder den Objekten bestückt und versiegelt, wobei die Verpackung bevorzugt durchlässig für das jeweilige Funktionsgas ist.

Die Eigenschaften der Verpackungen und/oder Verpackungsmaterialien sind bevorzugt in den Normen DIN EN ISO 11607-1 und 11607-2 oder DIN EN 868 geregelt. Diese Normen werden durch Bezugnahme vollumfänglich zum Gegenstand der vorliegenden Schrift gemacht.

Eine negative oder korrespondierende Ausbildung bezeichnet hierbei bevorzugt eine Ausgestaltung im Sinne eines "Schlüssels und eines Schlosses" oder einem "Stecker und einer Dose zur Aufnahme des Steckers".

Einzelne oder alle Darstellungen der im Nachfolgenden beschriebenen Figuren sind bevorzugt als Konstruktionszeichnungen anzusehen, d.h. die sich aus der bzw. den Figuren ergebenden Abmessungen, Proportionen, Funktionszusammenhänge und/oder Anordnungen entsprechen bevorzugt genau oder bevorzugt im Wesentlichen denen der erfindungsgemäßen Vorrichtung bzw. des erfindungsgemäßen Produkts.

Weitere Vorteile, Ziele und Eigenschaften vorliegender Erfindung werden anhand nachfolgender Beschreibung anliegender Zeichnungen erläutert, in welchen beispielhaft erfindungsgemäße Wärmflaschensicherheitsverschlüsse und deren Komponenten dargestellt sind. Elemente der erfindungsgemäßen Einrichtungen, welche in den Figuren wenigsten im Wesentlichen hinsichtlich ihrer Funktion übereinstimmen, können hierbei mit gleichen Bezugszeichen gekennzeichnet sein, wobei diese Bauteile bzw. Elemente nicht in allen Figuren beziffert oder erläutert sein müssen. Nachfolgend wird die Erfindung rein beispielhaft anhand der beigefügten Figuren beschrieben.

Die Verwendung der Wörter "im Wesentlichen" definiert bevorzugt in allen Fällen, in denen diese Wörter im Rahmen der vorliegenden Erfindung verwendet werden eine Abweichung im Bereich von 1%-30%, insbesondere von 1%-20%, insbesondere von 1%-10%, insbesondere von 1%-5%, insbesondere von 1%-2%, von der Festlegung, die ohne die Verwendung dieser Wörter gegeben wäre.

Darin zeigen:
- Fig. 1a: eine schematische Seitenansicht einer ersten beispielhafte Ausführungsform eines erfindungsgemäßen Handbetätigungselements;
- Fig. 1b: eine schematische Draufsicht auf eine erste beispielhafte Ausführungsform eines Verschlussteil;
- Fig. 1c: eine schematische Seitenansicht eines ersten beispielhaften mehrteiligen Wärmflaschensicherheitsverschlusses;
- Fig. 1d: eine schematische Draufsicht auf einen ersten beispielhaften mehrteiligen Wärmflaschensicherheitsverschluss;
- Fig. 2a: eine schematische Seitenansicht einer zweiten beispielhafte Ausführungsform eines erfindungsgemäßen Handbetätigungselements und eines zweiten erfindungsgemäßen Verschlussteils in einer entkoppelten Anordnung;
- Fig. 2b: eine schematische Draufsicht auf eine zweite beispielhafte Ausführungsform eines mehrteiligen Wärmflaschensicherheitsverschlusses;
- Fig. 2c: eine schematische Draufsicht auf die Unterseite eines zweiten beispielhaften Handbetätigungselements;
- Fig. 2d: eine schematische Draufsicht auf einen zweiten beispielhaften Verschlussteil;
- Fig. 3a: eine schematische Seitenansicht einer dritten beispielhafte Ausführungsform eines Handbetätigungselements und eines zweiten Verschlussteils in einer gekoppelten Anordnung;
- Fig. 3b: eine schematische Seitenansicht einer dritten beispielhafte Ausführungsform eines Handbetätigungselements und eines zweiten Verschlussteils in einer entkoppelten Anordnung;
- Fig. 3c: eine weitere schematische Seitenansicht des dritten beispielhaften Handbetätigungselements, wobei das Handbetätigungselement gegenüber der in Fig. 3b gezeigten Ansicht um 90° gedreht dargestellt ist;
- Fig. 3d: eine schematische Draufsicht auf das dritte beispielhaft dargestellte Handbetätigungselement, wobei das Handbetätigungselement entsprechend der Fig. 3b ausgerichtet ist;
- Fig. 4: eine Wärmflasche mit dem rein beispielhaft in Fig. 1c gezeigten mehrteiligen Wärmflaschensicherheitsverschluss;
- Fig. 5a-5j: verschiedene schematische Darstellungen von bevorzugten Querschnitten eines vom Handbetätigungselement oder vom Verschlussteil hervorstehenden Kraftübertragungselement; und
- Fig. 6: eine verschlossene Wärmflasche, die von Dritten nicht unbefugt geöffnet werden kann.
- Fig. 7a: eine perspektivische Darstellung eines bevorzugten Verschlussteils;
- Fig. 7b: eine perspektivische Darstellung eines zu dem in Fig. 7a gezeigten Verschlussteil passenden Handbetätigungselements und
- Fig. 8a-c: schematische Querschnittsdarstellungen durch erfindungsgemäße Verschlussteile;
- Fig. 8d-f: schematische Querschnittsdarstellungen durch erfindungsgemäße Handbetätigungselemente;
- Fig. 9a: eine Draufsicht auf ein Beispiel eines erfindungsgemäßen Verschlussteils;
- Fig. 9b: eine perspektivische Unteransicht des aus Fig. 9a bekannten Verschlussteils;
- Fig. 9c: eine Unteransicht des in Fig. 9b perspektivisch dargestellten Verschlussteils;
- Fig. 10a: eine Schnittdarstellung gemäß dem in Fig. 9a mit B-B gekennzeichneten Schnitt,
- Fig. 10b: eine Schnittdarstellung gemäß dem in Fig. 9a mit A-A gekennzeichneten Schnitt,
- Fig. 11a-b: zwei schematische und rein beispielhafte Darstellungen einer ersten Ausführungsform eines Verschlussteilanbringungselement;
- Fig. 11c-d: zwei schematische und rein beispielhafte Darstellungen gemäß denen das Verschlussteilanbringungselement aus den Figuren 11a-b mit einem Wärmflaschenverschlussteil gekoppelt ist;
- Fig. 11e-f: zwei schematische und rein beispielhafte Darstellungen einer zweiten Ausführungsform eines Verschlussteilanbringungselement;
- Fig. 11g: eine schematische und rein beispielhafte Darstellung gemäß der das Verschlussteilanbringungselement aus den Figuren 11e-f mit einem Wärmflaschenverschlussteil gekoppelt ist;
- Fig. 11h: drei schematische und rein beispielhafte Darstellungen weiterer möglicher Ausführungsformen des Verschlussteilanbringungselements;
- Fig. 11i-k: zwei schematische und rein beispielhafte Darstellungen eines Wärmflaschenverschlussteils mit einer zentralen Kopplungsstelle zum Ankoppeln eines Verschlussteilanbringungselements;
- Fig. 111-m: zwei schematische und rein beispielhafte Darstellungen eines weiteren Verschlussteilanbringungselements, das bevorzugt zumindest im Bereich eines Kopplungsdorns negativ zur Kopplungsstelle des in den Figuren 11i-k gezeigten Wärmflaschenverschlussteils ausgebildet ist

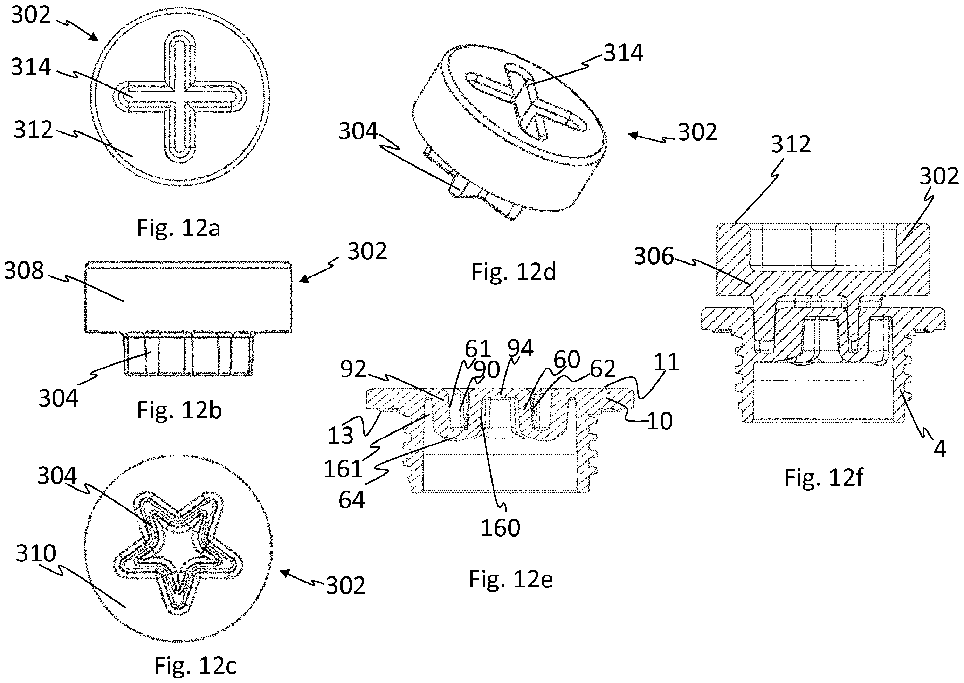

- Fig. 12a: eine schematische und rein beispielhafte Darstellung einer Draufsicht auf ein als Adapter bzw. Zwischenstück ausgebildetes Verschlussteilanbringungselements;
- Fig. 12b: eine schematische und rein beispielhafte Darstellung einer Seitenansicht des in Fig. 12a gezeigten Verschlussteilanbringungselements;
- Fig. 12c: eine schematische und rein beispielhafte Darstellung einer Ansicht von Unten des in Fig. 12a gezeigten Verschlussteilanbringungselements;
- Fig. 12d: eine schematische und rein beispielhafte perspektivische Darstellung des in den Figuren 12a-c gezeigten Verschlussteilanbringungselements;
- Fig. 12e: eine exemplarische Schnittdarstellung eines Wärmflaschenverschlussteils;
- Fig. 12f: eine exemplarische Schnittdarstellung des in Fig. 12d gezeigten Verschlussteilanbringungselements und des in Fig. 12e gezeigten Wärmflaschenverschlussteils in einer gekoppelten Anordnung; und

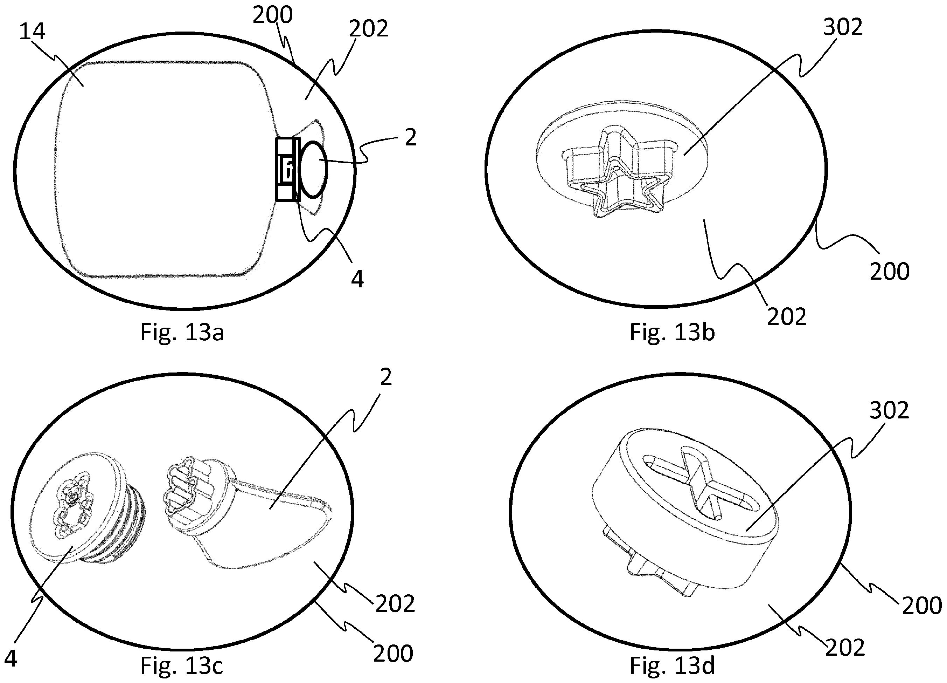

- Fig. 13a-d: Verschiedene Lose, wobei jedes Los eine Verpackung und ein erfindungsgemäßes Objekt enthält, das zumindest keimreduziert in der Verpackung vorgehalten bzw. angeordnet ist.

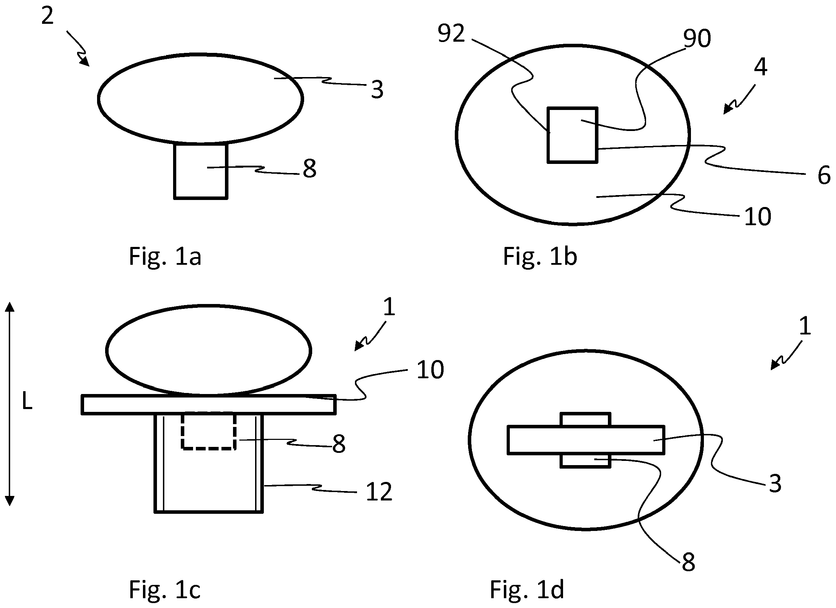

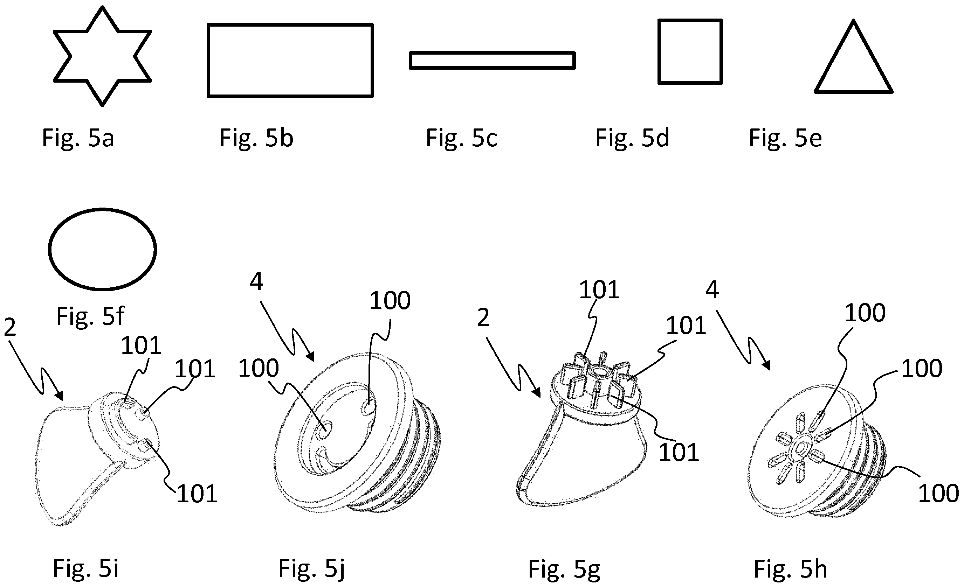

In Fig. 1a ist ein Handbetätigungselement 2 gezeugt. Die Form des Elements zur händischen Kontaktierung und Krafteinleitung 3 ist hierbei oval dargestellt kann jedoch auch rund, rechteckig oder in einer anderen beliebigen Form ausgebildet sein. Das Bezugszeichen 8 kennzeichnet das zweite Kopplungselement, das in der dargestellten Ausführungsform zapfenartig ausgebildet ist. Beispiele für die Querschnittsform des zapfenartigen zweiten Kopplungselements 8 sind in den Fig. 5a bis 5f gezeigt. Die durch die Figuren 5a bis 5e gezeigten Querschnitte ermöglichen einen Formschluss mit dem korrespondierend bzw. negativ ausgebildeten ersten Kopplungselement 6 des Verschlussteils 4 (vgl. Fig. 1b und 1c). Der in Fig. 5f gezeigte Querschnitt erfordert einen Kraftschluss bzw. ein Einklemmen des zapfenartig ausgebildeten zweiten Kopplungselements 8 durch das negativ bzw. korrespondierend ausgebildete erste Kopplungselement 6 des Verschlussteils 4. Das Bezugszeichen 10 kennzeichnet einen bevorzugt umlaufenden Kragen, der besonders bevorzugt als Dichtmittel dient bzw. ein Dichtmittel aufweist und/oder als Anschlag fungiert.

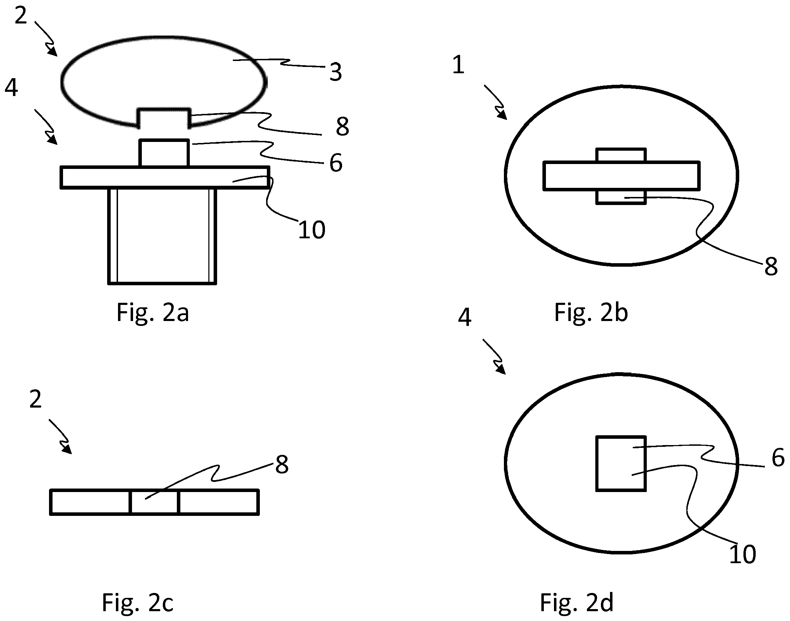

In den Fig. 2a bis 2d ist ein Ausführungsbeispiel gezeigt, gemäß dem das erste Kopplungselement 6, das am Verschlussteil 4 angeordnet oder ausgebildet ist, zapfenartig ausgebildet ist. Das am Element zur händischen Kontaktierung 3 und Kraftaufbringung bzw. -einleitung ausgebildete oder angeordnete zweite Kopplungselement 6 ist hierbei negativ bzw. korrespondierend zum ersten Kopplungselement 6 ausgebildet.

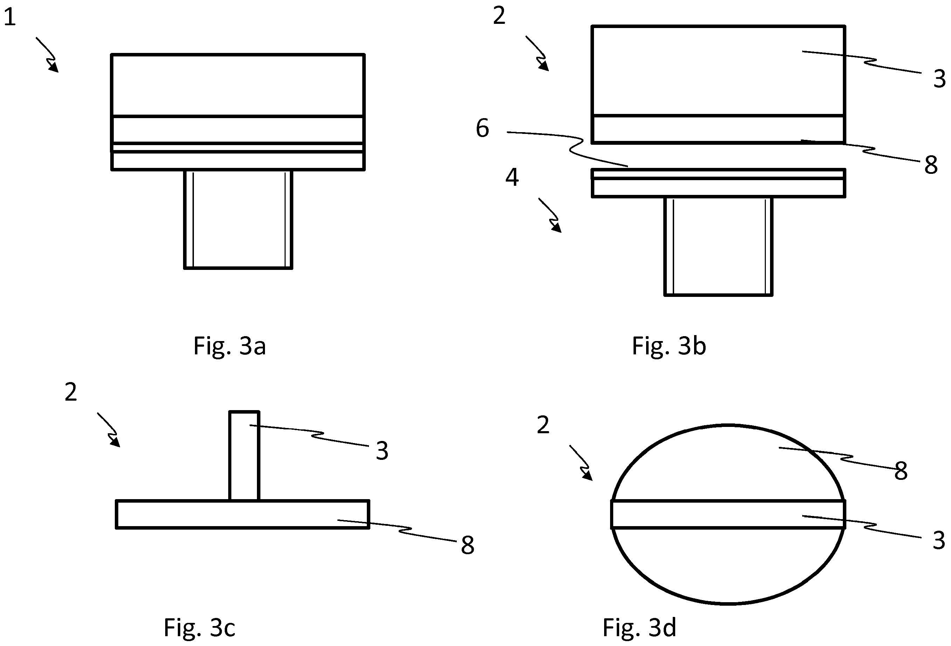

Die Fig. 3a-3d zeigen schematisch eine Ausführungsform, gemäß der das erste Kopplungselement 6 und das zweite Kopplungselement 8 eben bzw. im Wesentlichen eben ausgebildet sind. Es ist hierbei denkbar, dass das erste Kopplungselement 6 und das zweite Kopplungselement 8 jeweils durch einen Magneten ausgebildet werden. Alternativ oder zusätzlich ist denkbar, dass das erste Kopplungselement 6 und das zweite Kopplungselement 8 einen Klettverschluss ausbilden.

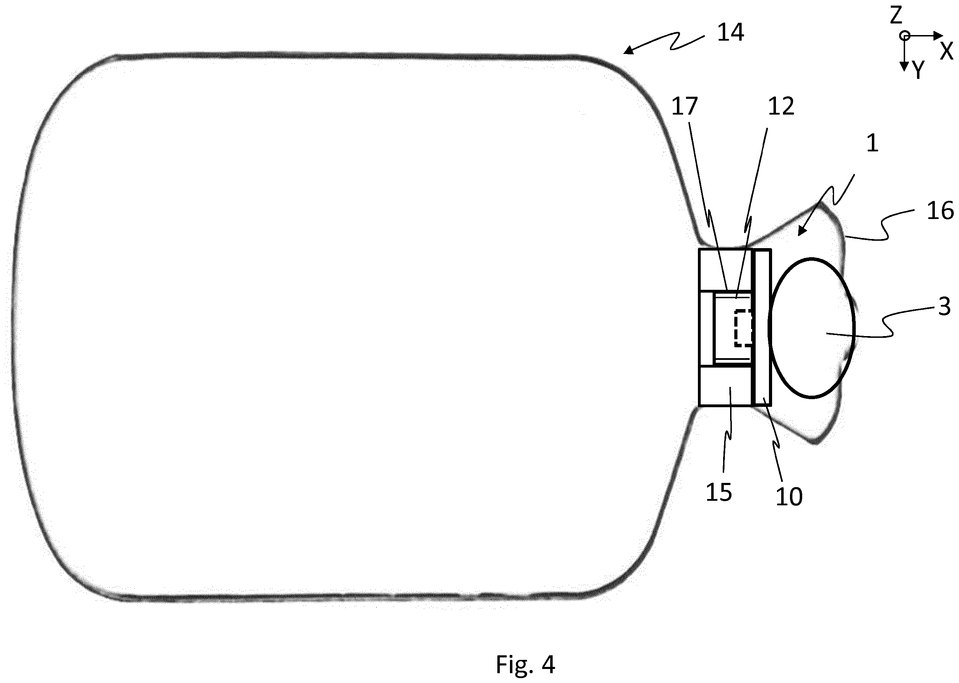

Fig. 4 zeigt eine Wärmflasche 14 mit einem Beispiel eines mehrteiligen Wärmflaschensicherheitsverschluss 1, insbesondere dem in Fig. 1c gezeigten mehrteiligen Wärmflaschensicherheitsverschluss.

Die Figur 5i zeigt ein Handbetätigungselement 2, das korrespondierend zu dem Verschlussteil 4 aus Fig. 5j ausgebildet ist. Ebenso zeigt Fig. 5g ein Handbetätigungselement 2, das korrespondierend zu dem durch Fig. 5h gezeigten Verschlussteil 4 ausgebildet ist. In beiden Fällen weisen das erste Kraftübertragungselement 6 und das zweite Kraftübertragungselement 8 jeweils eine Vielzahl, insbesondere mindestens 2, mindestens 3, mindestens 4, mindestens 5, mindestens 6, mindestens 7, mindestens 10 oder mindestens 15, von zumindest abschnittsweise und bevorzugt vollständig voneinander beabstandete Teilkraftübertragungselemente 100, 101 auf, wobei die Teilkraftübertragungselemente 100 des ersten Kraftübertragungselements 6 korrespondierend, insbesondere negativ, zu den Teilkraftübertragungselementen 101 des zweiten Kraftübertragungselements 8 ausgebildet sind. Die Teilkraftübertragungselemente 100 werden bevorzugt als Bestandteil des Verschlussteils 4 und die Teilkraftübertragungselemente 101 werden bevorzugt als Bestandteil des Handbetätigungselements 2 erzeugt, insbesondere in einem Spritzgussprozess.

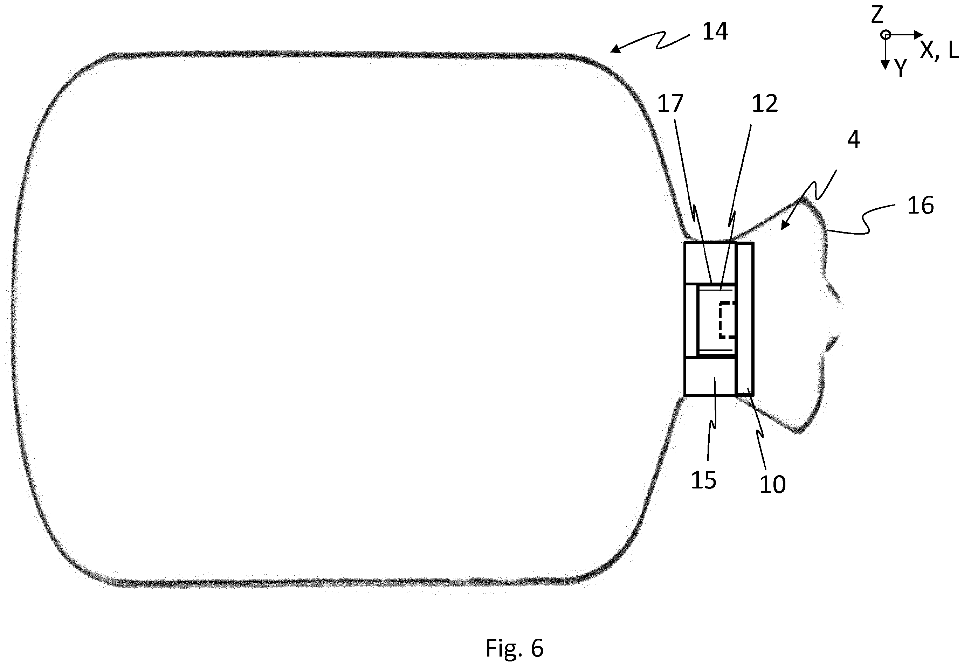

Fig. 6 zeigt eine verschlossene Wärmflasche 14, die von Dritten nicht unbefugt geöffnet werden kann, solange das hier nicht dargestellte Handbetätigungselement 2 dem oder den Dritten nicht zugänglich gemacht wird. Das Verschlussteil 4 ist hierbei so gestaltet, dass es für eine Person (nur mit den Händen) nicht möglich ist die Wärmflasche zu öffnen, da die zum Rotieren bzw. Aufdrehen des Verschlussteils 4 erforderlichen Kräfte nicht in das Verschlussteil 4 eingeleitet werden können bzw. von einer Person nicht aufgebracht werden können.

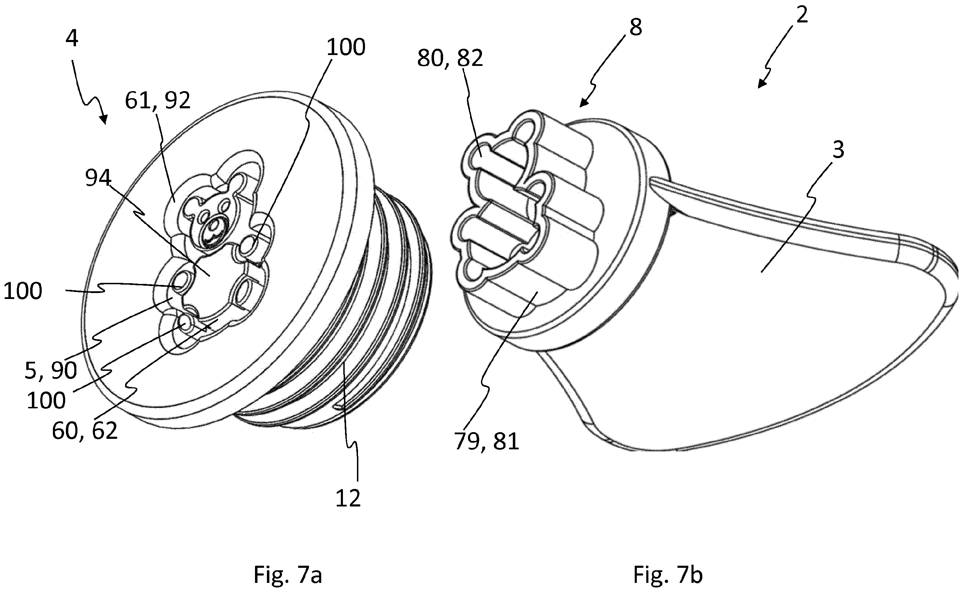

In Fig. 7a ist eine perspektivische Ansicht eines erfindungsgemäßen Verschlussteils 4 gezeigt. Es ist ersichtlich, dass das erste Kraftübertragungselement 6 zumindest abschnittsweise, insbesondere mehrheitlich oder vollständig, gebogen ausgebildet ist.

Bevorzugt wird zumindest ein Teil eines Eindringbereichs des Verschlussteils, in den das zweite Kraftübertragungselement 8 einführbar ist, durch eine zumindest abschnittsweise und bevorzugt mehrheitlich und besonders bevorzugt vollständig den Eindringbereich 90 umschließende Wandung 92 begrenzt.

Gemäß einer bevorzugten Ausführungsform wird das erste Kraftübertragungselement 8 durch eine Wandung, insbesondere eine umschließende Wandung 92, ausgebildet, wobei die Wandung 92 einen Eindringbereich 92, insbesondere eine Ausnehmung 5, begrenzt, wobei der Eindringbereich 92, insbesondere die Ausnehmung 5, bevorzugt eine streifenförmige, insbesondere eine umlaufende, Kontur beschreibt, wobei sich die Ausnehmung 5 orthogonal zur Kontur bevorzugt weniger als 10mm, insbesondere weniger als 9mm oder weniger als 8mm oder weniger als 7mm oder weniger als 6mm oder weniger als 5mm oder weniger als 4mm oder weniger als 3mm , insbesondere zwischen 0,5mm und 2,9mm, wie z.B. zwischen 1mm und 2 mm, weit erstreckt. Bevorzugt ist das erste Kraftübertragungselement 8 derart gestaltet, dass es über eine äußere Kraftübertragungsfläche 61 und/oder über eine innere Kraftübertragungsfläche 62 mit dem zweiten Kraftübertragungselement 6 des Verschlussteils 4 zusammenwirken kann.

Die Ausnehmung 5 ist bevorzugt so schmal oder groß und bevorzugt so gebogen gestaltet, dass nicht zur Betätigung des Verschlussteils 4 gedachte Einrichtungen, wie Messer oder Münzen, nicht zum Öffnen des Verschlusses verwendet werden können.

In Fig. 7b ist eine perspektivische Ansicht eines erfindungsgemäßen Handbetätigungselements 4 gezeigt. Es ist ersichtlich, dass das zweite Kraftübertragungselement 8 zumindest abschnittsweise, insbesondere mehrheitlich oder vollständig, gebogen ausgebildet ist.

Bevorzugt weist das zweite Kraftübertragungselement 8 zumindest abschnittsweise eine außenliegende Wandung 79 auf, die mit der umschließenden Wandung 92 des Eindringbereichs 90 koppelbar ist.

Gemäß einer bevorzugten Ausführungsform der vorliegenden Erfindung wird das zweite Kraftübertragungselement 8 durch eine Wandung ausgebildet, wobei die Wandung weniger als 10 mm, insbesondere weniger als 9mm oder weniger als 8 mm oder weniger als 7mm oder weniger als 6mm oder weniger als 5mm oder weniger als 4mm oder weniger als 3mm , insbesondere zwischen 0,5mm und 2,9mm, wie z.B. zwischen 1mm und 2 mm, dick ist. Bevorzugt ist das zweite Kraftübertragungselement 8 derart gestaltet, dass es über eine äußere Kraftübertragungsfläche 81 der außenliegenden Wandung und/oder über eine innere Kraftübertragungsfläche 82 einer innenliegenden Wandung 80 mit dem ersten Kraftübertragungselement 6 des Verschlussteils 4 zusammenwirken kann.

Bevorzugt wirkt zumindest die äußere Kraftübertragungsfläche 81 des zweiten Kraftübertragungselements 8 mit der äußere Kraftübertragungsfläche 61 des ersten Kraftübertragungselements 6 zusammen und/oder es wirkt zumindest die innere Kraftübertragungsfläche 82 des zweiten Kraftübertragungselements 8 mit der inneren Kraftübertragungsfläche 62 des ersten Kraftübertragungselements 6 zusammen. Wobei selbstverständlich denkbar ist, dass der Verschlussteil 4 nur eine äußere Kraftübertragungsfläche 62, 61 aufweist. Entsprechend dem Handbetätigungselement 2.

Wobei eine äußere Kraftübertragungsfläche der den Eindringbereich 90 umschließend begrenzenden Wandung 92 bevorzugt weiter als eine äußere Kraftübertragungsfläche der Erhebung von einer Rotationsachse des Verschlussteils oder Handbetätigungselements beabstandet ist, wobei beim Öffnen und Schließen einer Wärmfasche das jeweilige Bauteil um die Rotationsachse rotiert wird. Äußere Kraftübertragungsflächen 61, 62 liegen auf der Seite (Außenseite) des Verschlussteils 2, der wenn der Verschlussteil 2 in die Wärmflasche geschraubt ist zugänglich ist und innere Kraftübertragungsflächen 160, 161 liegen auf der Seite (Innenseite) des Verschlussteils 2, die bei in die Wärmflasche eingeschraubtem Verschlussteil 2 unzugänglich ist, d.h. innerhalb des Gewindestücks 15.

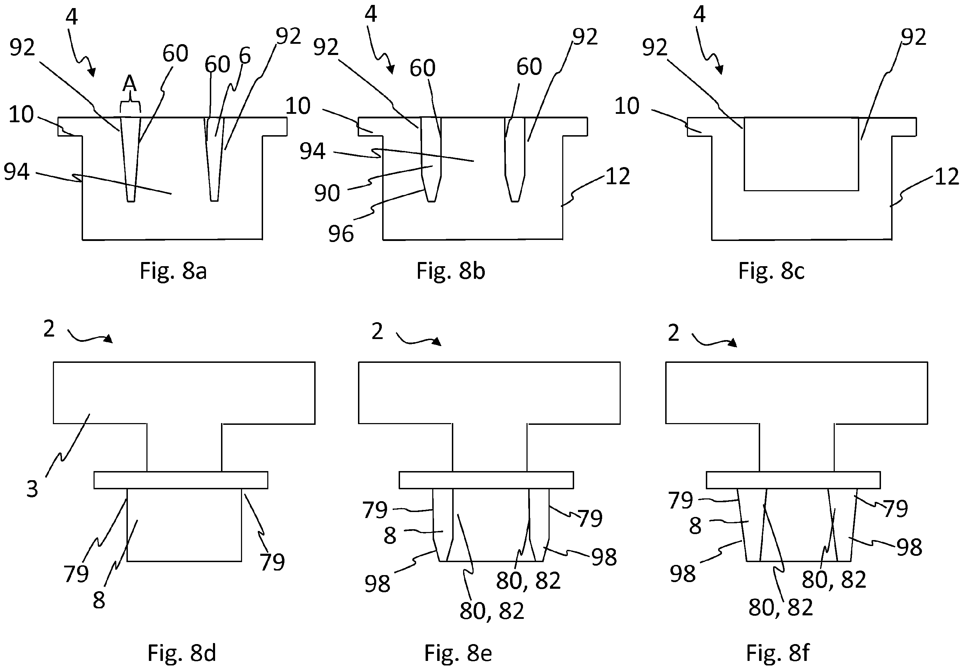

Fig. 8a-8c zeigen drei verschiedene Darstellungen von Beispielen für erfindungsgemäße Verschlussteile 4. Der Eindringbereich 90 der einzelnen Verschlussteile 4 ist jedoch verschieden. Fig. 8a und 8b zeigen Verschlussteile mit einer Erhebung 94 hingegen weist Fig. 8c keine solche Erhebung somit auch keine äußere umlaufende Wandung 60 der Erhebung 94 auf. Gemäß den Figuren 8a und 8b kann der Eindringbereich teilweise, abschnittsweise, vollständig oder mehrheitlich verjüngend ausgebildet sein. Es ist jedoch ebenfalls denkbar, dass der Eindringbereich durch zueinander bevorzugt parallel ausgerichtete oder geneigt ausgerichtete Wandungen 92, 60 begrenzt wird. Das Bezugszeichen 96 kennzeichnet einen sich verjüngenden Anteil des Eindringbereichs 90.

Die durch die Figuren 8d-8f dargestellten beispielhaften Handbetätigungselemente 2 weisen jeweils ein Griffteil 3 auf. Ferner weisen die durch die Figuren 8e und 8f dargestellten Beispiele innenliegende Wandungen 80 zur Ausbildung von Kraftübertragungsflächen 82 auf. Es ist somit denkbar, dass das zweite Kraftübertragungsmittel 8 (des Handbetätigungselements 2) nur über die innenliegende Wandung 80 Kräfte bzw. Momente zum Eindrehen oder Herausdrehen des Verschlussteils 4 in das erste Kraftübertragungselement 6 (des Verschlussteils 4) einleitet. Alternativ ist jedoch denkbar, dass die Kräfte über die innenliegende Oberfläche 80, 82 und die außenliegende Wandung 79 übertragen werden. Ferner ist denkbar, dass die Kräfte und Momente lediglich über die außenliegende Oberfläche übertragen werden. Fig. 8d zeigt z.B. ein Handbetätigungselement 2, das keine innenliegende Oberfläche zur Übertragung von Kräften und Momenten aufweist und somit ausschließlich über die außenliegende Oberfläche 79 Kräfte und Momente überträgt.

Weiterhin ist denkbar, dass jedes der gezeigten Handbetätigungselemente 2 mit einem Verschlussteil gemäß Fig. 8c zusammen wirken kann. Ferner ist denkbar, dass Handbetätigungselemente gemäß der Figuren 8e und 8f mit Verschlussteilen 4 gemäß den Figuren 8a und 8b zusammen wirken können.

Bevorzugt werden das Verschlussteil 4 und das Handbetätigungselement 2 somit durch das Zusammenwirken des ersten Kraftübertragungselements 6 und des zweiten Kraftübertragungselements 8 in einem gekoppelten Zustand, insbesondere kraftschlüssig oder formschlüssig oder feldschlüssig, gehalten werden. Bevorzugt ist zwischen dem ersten Kraftübertragungselement 6 und dem zweiten Kraftübertragungselement 8 eine Punktpressung oder eine Linienpressung oder eine Flächenpressung erzeugbar, durch welche bevorzugt der Kraftschluss erzeugt wird. Dies ist vorteilhaft, da beim Herausdrehen des Verschlussteils 4 mittels des Handbetätigungselements 2 das Verschlussteil 4 zugbelastet wird und dadurch im Moment des vollständigen Herausschraubens unmittelbar an dem Handbetätigungselement 2 gehalten wird. Dies ist äußerst hilfreich, da der Nutzer so ein Objekt in der Hand hält und dieses leichter verstauen kann. Ferner fällt das Verschlussteil 4 im Moment des Herausschraubens nicht nach unten, wodurch die Gefahr des Verlorengehens bestehen würde.

Gemäß einer weiteren bevorzugten Ausgestaltung beträgt der Abstand A zwischen der Erhebungsoberfläche 62 der Wandung 60 der Erhebung 94 (oder Erhebungswandung 60) und der äußere Kraftübertragungsfläche 61 der umschließenden Wandung 92 weniger als 12 mm, insbesondere weniger als 11 mm oder weniger als 9 oder weniger als 8 mm oder weniger als 7 oder weniger als 6 mm oder weniger als 5 mm oder weniger als 4 mm oder weniger als 3 mm oder weniger als 2 mm, insbesondere weniger als 1,5 mm oder 1mm. Bevorzugt weisen die Erhebung 94 und die umschließende Wandung 92 dieselbe Höhe oder im Wesentlichen dieselbe Höhe auf.

Das zweite Kraftübertragungselement 8 ist bevorzugt über die Wandung 60 der Erhebung 94 und/oder über die äußere Kraftübertragungsfläche 61 der umschließenden Wandung 92 mit dem ersten Kopplungselement 6 koppelbar.

Als äußere Kraftübertragungsfläche 61 wird hierbei bevorzugt eine Oberfläche der Wandung 92 bezeichnet, die in Richtung Drehzentrum des Verschlussteils gerichtet ist und als äußere Kraftübertragungsfläche 62 wird hierbei bevorzugt eine Oberfläche der Wandung 60 der Erhebung 94 bezeichnet, die nach außen (insbesondere radial nach außen) gerichtet ist und von der äußeren Kraftübertragungsfläche 61 zumindest abschnittsweise umgeben wird.

Die Erhebung 94 ist bevorzugt derart gestaltet, dass die Einleitung eines Drehmoments von 2Nm +/-0,1 Nm in das Verschlussteil 2 zum wasserdichten Verschließen und zum Öffnen einer Wärmflasche eine Kraftbeaufschlagung der Erhebung 94 mit einer Kraft von mehr als 50 N, insbesondere von mehr als 60 N oder von mehr als 70 N, erfordert, wobei die Erhebung 94 gemäß einer bevorzugten Ausführungsform, insbesondere gemäß dieser Ausführungsform in Längsrichtung L des Verschlussteils 2 nicht oder nur geringfügig, insbesondere bis zu 7mm oder bis zu 6mm oder bis zu 5mm oder bis zu 4mm oder bis zu 3mm oder bis zu 2mm oder bis zu 1mm, über die den Kragen 10 in Längsrichtung L begrenzende Kragenoberfläche 13 hervorsteht. Zusätzlich oder alternativ weist das zweite Kraftübertragungselement 8 zumindest abschnittsweise eine außenliegende Wandung 79 auf, die mit der umschließenden Wandung 92 des Eindringbereichs 90 koppelbar ist, wobei bevorzugt der Eindringbereich 90 zusätzlich zur umschließenden Wandung 92 ebenfalls durch mindestens eine Wandung 60 von mindestens einer im durch die umschließende Wandung 92 begrenzten Bereich ausgebildeten Erhebung 94, insbesondere einem zapfenartigen oder säulenartigen Element, begrenzt wird.

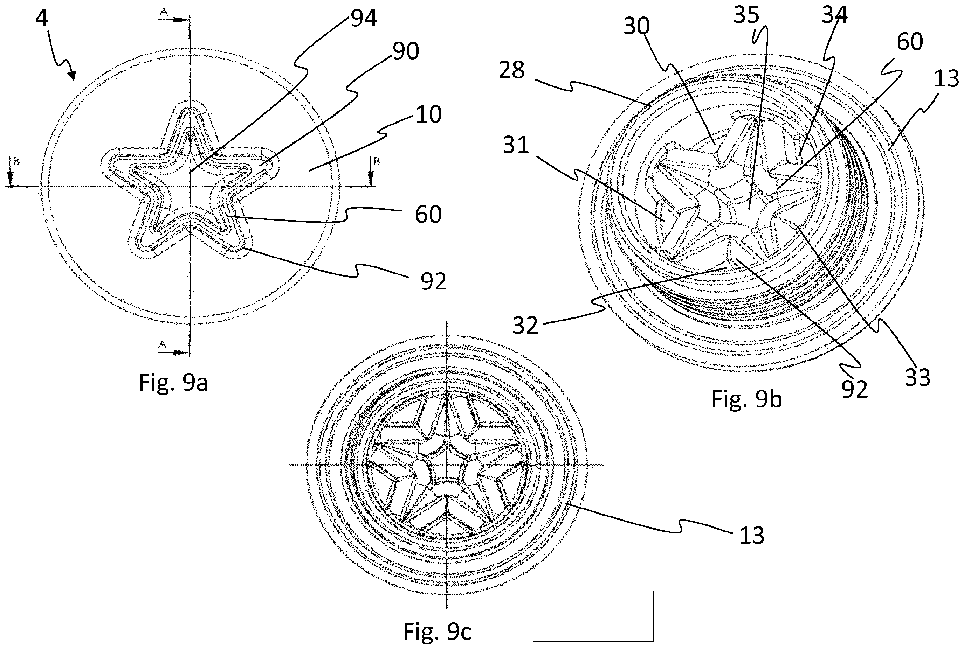

Fig. 9a zeigt eine Draufsicht auf ein Beispiel eines erfindungsgemäßen Verschlussteils 2. Das Verschlussteil 4 weist einen Eindringbereich 90 zur Aufnahme des Handbetätigungselements 2 auf. Der Eindringbereich 90 wird gemäß dieser speziellen Ausführungsform durch eine den Eindringbereich umschließende Wandung 92 einerseits begrenzt. Andererseits wird der Eindringbereich 90 durch eine Wandung 60 begrenzt, durch die eine Erhebung 94 ausgebildet wird. Es ist hierbei jedoch alternativ ebenfalls denkbar, dass die Wandung 60 und somit die Erhebung 90 nicht ausgebildet ist. Das Bezugszeichen 10 kennzeichnet den Kragen des Verschlussteils 4.

Fig. 9b zeigt eine perspektivische Unteransicht des aus Fig. 9a bekannten Verschlussteils 4. Es kann der Darstellung entnommen werden, dass eine Vielzahl, insbesondere mehr als zwei Hohlräume 30-35 bevorzugt mehr als 3 und besonders bevorzugt mehr als 4, wie z.B. 5 oder 6, zwischen der Wandung 92 und einer das Gewinde aufweisenden Umfangswandung 28 ausgebildet sind. Die den Eindringbereich 90 umschließende Wandung 92 ist somit auf der Verschlussteilunterseite zumindest abschnittsweise zu einer das Gewinde aufweisenden Umfangswandung 28 beabstandet, wodurch die mehreren voneinander abgegrenzten Freiräume 30-34 zwischen der das Gewinde aufweisenden Umfangswandung 28 und der den Eindringbereich 90 umschließenden Wandung 92 ausgebildet sind oder ein umlaufender Freiraum zwischen der umschließenden Wandung 92 und der das Gewinde aufweisenden Umfangswandung 28 ausgebildet ist. Bevorzugt ergeben sich die Freiräume 30-34 oder der Freiraum 35 aus einer maximalen Wandstärker der umschließenden Umfangswandung 28, wobei die maximale Wandstärke weniger als 5mm beträgt, insbesondere weniger als 4mm beträgt oder weniger als 3mm beträgt oder weniger als 2mm beträgt. Zusätzlich oder alternativ wird der Kragen 10 durch eine Kragenwandung ausgebildet, die in Längsrichtung L mindestens eine Dicke von 1mm, oder von 1,5mm oder von 2mm und bevorzugt von mindesten 2,5mm oder von mindestens 2,8 mm oder von mindestens 3,0mm oder von mindestens 3,3mm oder von mindestens 3,45mm oder von mindestens 3,55mm oder von mindestens 3,6mm oder von mindestens 3,65mm oder von mindestens 3,7mm oder von mindestens 3,75 mm aufweist.

In Fig. 9b und in Fig. 9c ist ferner eine Dichtung 13 bzw. ein Dichtungselement 13 dargestellt. Bevorzugt ist die Dichtung bzw. das Dichtungselement Bestandteil des Kragens 10, insbesondere einstückig damit ausgebildet. Die Dichtung 13 bzw. das Dichtungselement 13 wirkt zum Abdichten der Wärmflasche bevorzugt mit einem Gewindestück 15 pressend zusammen oder wirkt zum Abdichten mit einem Anteil des den Wärmflaschenkörper bildenden Materials pressend zusammen. Bevorzugt ist das Gewindestück 15 mit dem den Wärmflaschenkörper bildenden Material zumindest abschnittsweise umgeben, insbesondere ist das Gewindestück 15 bevorzugt von dem Material zumindest abschnittsweise umspritzt. Besonders bevorzugt wirkt die Dichtung 13 bzw. das Dichtungselement 13 zum Abdichten mit dem das Gewindestück umgebenden Material des Wärmflaschenkörpers zusammen.

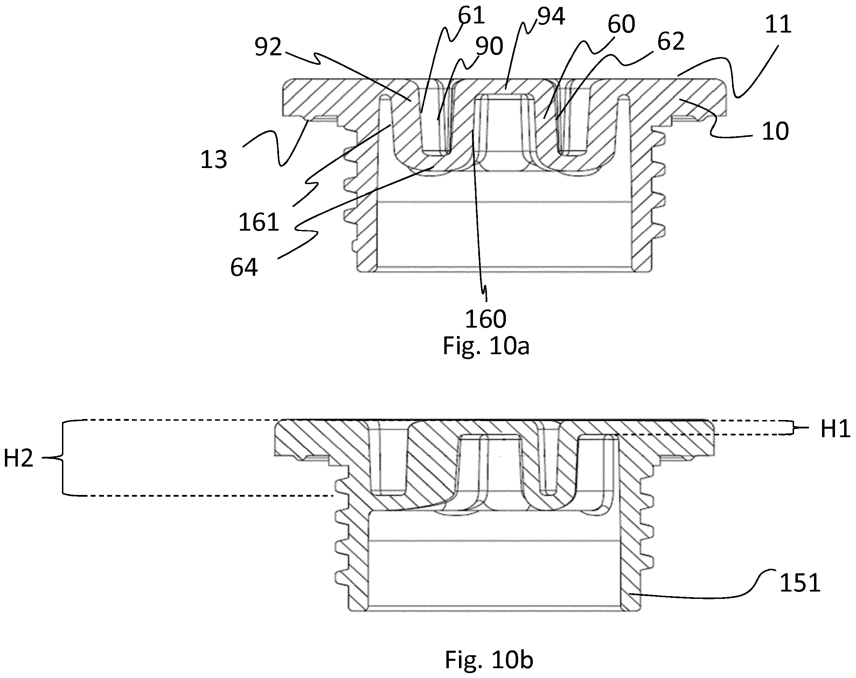

Fig. 10a zeigt einen Querschnitt durch den in Fig. 9a gezeigten Verschlussteil 4. Es ist erkennbar, dass der Eindringbereich 90 in dieser Ausführungsform durch die den Eindringbereich außen umschließende Wandung 92 begrenz wird. Diese Wandung ist in dieser Ausführungsform und bevorzugt in allen anderen Ausführungsformen die einen Eindringbereich 90 aufweisen mindestens 0,8mm dick und bevorzugt mindestens 1mm dick und besonders bevorzugt mindestens 1,2mm dick oder mindestens 1,5mm dick oder mindestens 1,8mm dick oder mindestens 2mm dick. Bevorzugt ist die Wandung 92 höchstens 5mm dick und besonders bevorzugt höchstens 3mm dick oder höchstens 2,5mm dick oder höchstens 2mm dick oder höchstens 1,5mm dick. Die äußere Oberfläche 61 der Wandung 92 ist in der Querschnittsdarstellung gegenüber der Oberfläche 11 bevorzugt geneigt ausgebildet, insbesondere in einem Winkel zwischen 60° und 90° ausgebildet und bevorzugt in einem Winkel zwischen 70°und 89° oder zwischen 80° und 88° ausgebildet.

Die Wandung 60, welche die Erhebung 94 ausbildet, ist bevorzugt mindestens 0,8mm dick und bevorzugt mindestens 1mm dick und besonders bevorzugt mindestens 1,2mm dick oder mindestens 1,5mm dick oder mindestens 1,8mm dick oder mindestens 2mm dick. Bevorzugt ist die Wandung 60 höchstens 5mm dick und besonders bevorzugt höchstens 3mm dick oder höchstens 2,5mm dick oder höchstens 2mm dick oder höchstens 1,5mm dick. Die äußere Oberfläche 62 der Wandung 60 ist in der Querschnittsdarstellung gegenüber der Oberfläche 11 bevorzugt geneigt ausgebildet, insbesondere in einem Winkel zwischen 60° und 90° ausgebildet und bevorzugt in einem Winkel zwischen 70°und 89° oder zwischen 80° und 88° ausgebildet.

Bevorzugt ist die Wandung 92 mit der Wandung 60 unmittelbar oder über eine Verbindungswandung 64 bzw. einen Verbindungswandungsanteil 64 verbunden. Die Verbindungswandung 64 ist bevorzugt mindestens 0,8mm dick und bevorzugt mindestens 1mm dick und besonders bevorzugt mindestens 1,2mm dick oder mindestens 1,5mm dick oder mindestens 1,8mm dick oder mindestens 2mm dick. Bevorzugt ist die Verbindungswandung 64 höchstens 5mm dick und besonders bevorzugt höchstens 3mm dick oder höchstens 2,5mm dick oder höchstens 2mm dick oder höchstens 1,5mm dick.

Ferner weist die Wandung 92 eine innere Oberfläche 161 auf. Die innere Oberfläche 161 der Wandung 92 ist in der Querschnittsdarstellung gegenüber der Oberfläche 11 bevorzugt geneigt ausgebildet, insbesondere in einem Winkel zwischen 60° und 90° ausgebildet und bevorzugt in einem Winkel zwischen 70°und 89° oder zwischen 80° und 88° ausgebildet.

Ferner weist die Wandung 60 eine innere bzw. innenliegende Oberfläche 160 auf. Die innere Oberfläche 160 der Wandung 60 ist in der Querschnittsdarstellung gegenüber der Oberfläche 11 bevorzugt geneigt ausgebildet, insbesondere in einem Winkel zwischen 60° und 90° ausgebildet und bevorzugt in einem Winkel zwischen 70°und 89° oder zwischen 80° und 88° ausgebildet.

Das Bezugszeichen 151 kennzeichnet die Innenwandung des Gewindeabschnitts.

In Fig. 10b sind bevorzugte Beispiele für Abmessungen angegeben. Die Höhe H1 kennzeichnet hierbei bevorzugt die Dicke des mit 941 gekennzeichneten Wandungsanteils. Bevorzugt weist der Wandungsanteil 941 eine Höhe H1 von bevorzugt mindestens 0,8mm und bevorzugt mindestens 1mm und besonders bevorzugt mindestens 1,2mm oder mindestens 1,5mm oder mindestens 1,8mm oder mindestens 2mm auf. Bevorzugt weist der Wandungsanteil 941 höchstens eine Höhe H1 von 5mm und besonders bevorzugt von höchstens 3mm oder von höchstens 2,5mm oder von höchstens 2mm oder von höchstens 1,5mm auf.

Die Höhe H2 kennzeichnet hierbei bevorzugt die Höhe des Eindringbereichs 90. Bevorzugt weist der Eindringbereich 90 eine Höhe H2 von bevorzugt mindestens 0,8mm und bevorzugt mindestens 1mm und besonders bevorzugt mindestens 1,2mm oder mindestens 1,5mm oder mindestens 1,8mm oder mindestens 2mm oder mindesten 3mm oder mindestens 4mm oder mindestens 5mm auf. Bevorzugt weist der Eindringbereich 90 höchstens eine Höhe H2 von 15mm und besonders bevorzugt von höchstens 12mm oder von höchstens 10mm oder von höchstens 8mm oder von höchstens 5mm oder von höchstens 2mm auf.

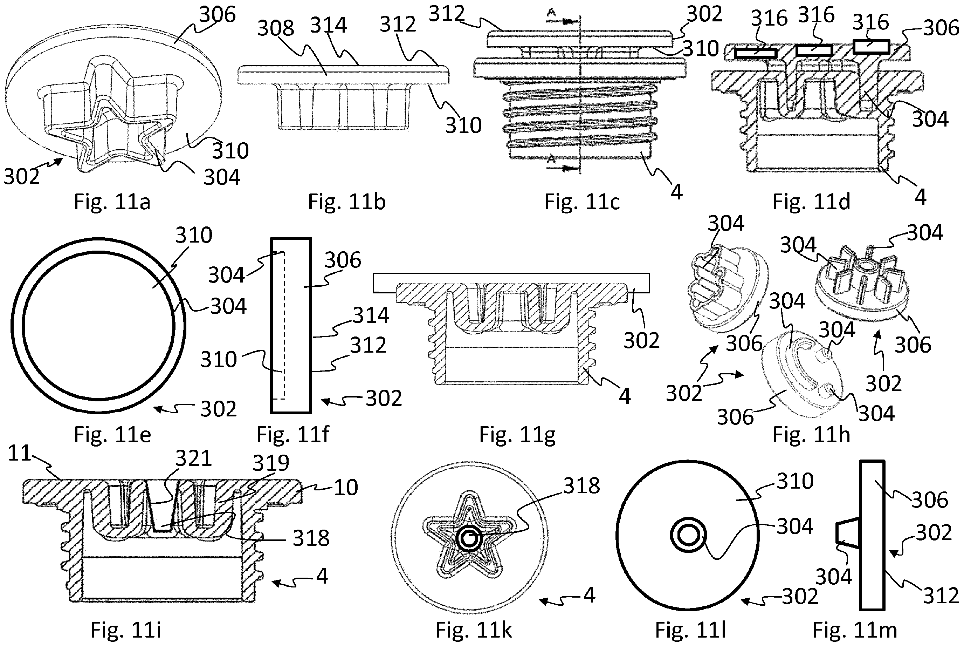

Fig. 11a zeigt eine schematische perspektivische Darstellung eines Verschlussteilanbringungselements 302 bzw. eines Wärmflaschensicherheitsverschluss-Verschlussteilanbringungselements 302. Das Verschlussteilanbringungselement 302 weist hierbei mindestens ein Funktionsmittel 306 auf, wobei das Funktionsmittel 306 einen Funktionsbereich 308 (vgl. Fig. 11b, 11m oder 12b) ausbildet, wobei der Funktionsbereich 308 zum zumindest abschnittsweisen Überlagern eines Kraftübertragungselements 6 eines Wärmflaschensicherheitsverschluss-Verschlussteils 4 ausgebildet ist. Bevorzugt ist an dem oder in dem (insbesondere als magnetischer Anteil) oder als Bestandteil des Funktionsmittels 306 ein oder genau ein oder mindestens ein Positioniermittel 304 angeordnet bzw. ausgebildet. Es ist hierbei jedoch ebenfalls denkbar, dass an dem Funktionsmittel 306 mehrere Positioniermittel 304 angeordnet oder ausgebildet sind. Das Positionierelement 304 ist bevorzugt auf einer ersten Seite 310 oder an einer ersten Seite des Funktionsmittels 306 angeordnet bzw. ausgebildet oder die Positionierelemente sind auf einer ersten Seite 310 oder an einer ersten Seite des Funktionsmittels 306 angeordnet bzw. ausgebildet sind. Das Funktionsmittel 306 wir auf der Seite, die der ersten Seite 310 gegenüberliegt, bevorzugt durch die zweite Seite 312 begrenzt. Die zweite Seite 312 bildet dabei eine bevorzugt abschnittsweise oder vollständig ebene Oberfläche aus. Es ist jedoch alternativ auch vorstellbar, dass die durch die zweite Seite 312 ausgebildete Oberfläche eine zumindest abschnittsweise dreidimensionale Struktur ausbildet (vgl. z.B. Fig. 11d oder 12a). Die zweite Seite 312 bildet dabei bevorzugt eine Kopplungsfläche 314 (Fig. 11a) oder Kopplungsstruktur 314 (Fig. 12a) aus.

Es ist hierbei ferner vorstellbar, dass das Funktionsmittel 306 magnetisch ausgestaltet ist bzw. mindestens oder genau ein magnetisches Element aufweist oder einen magnetischen Anteil aufweist. Bevorzugt ist dann über die zweite Seite 312 ein ebenfalls magnetisch wirksames Element mit dem Verschlussteilanbringungselement 302 magnetisch koppelbar. Zusätzlich oder alternativ ist denkbar, dass die zweite Seite 312 derart ausgebildet ist, dass ein weiteres Element stoffschlüssig oder formschlüssig oder kraftschlüssig daran lösbar oder unlösbar anordenbar ist.

Fig. 11b zeigt eine Seitenansicht der Darstellung von Fig. 11a.

Fig. 11c zeigt eine Seitenansicht eines Zusammenbaus eines erfindungsgemäßen Verschlussteils 4 bzw. Wärmflaschensicherheitsverschluss-Verschlussteils 4 mit einem weiteren Beispiel eines erfindungsgemäßen Verschlussteilanbringungselements 302. Es kann dieser Darstellung entnommen werden, dass das Funktionsmittel 306 bevorzugt auf einer zweiten Seite 312 (vgl. Fig. 11b), die von der ersten Seite 310 verschieden ist und die bevorzugt im Wesentlichen oder genau parallel zur ersten Seite 310 liegt, in einem Zustand, in dem das Verschlussteilanbringungselement 302 mit dem Wärmflaschensicherheitsverschluss-Verschlussteil 4 gekoppelt ist, sichtbar oder berührbar oder zugänglich ist. Die erste Seite 310 des Funktionsmittels 306 ist in dem Zustand, in dem das Verschlussteilanbringungselement 302 mit dem Wärmflaschensicherheitsverschluss-Verschlussteil 4 gekoppelt ist, bevorzugt zumindest teilweise unsichtbar oder unberührbar oder unzugänglich oder verdeckt.

Fig. 11d zeigt eine Schnittdarstellung des in Fig. 11c gezeigten Zusammenbaus. Der Schnitt verläuft dabei gemäß dem in Fig. 11c durch "A" gekennzeichneten Schnittverlauf. Es kann dieser Darstellung entnommen werden, dass das Funktionsmittel 306 Elektronikmittel 316 aufweisen kann. Es ist hierbei im Sinne der vorliegenden Erfindung, dass bevorzugt ein, genau ein oder mindestens ein Elektronikmittel 316 vorgesehen ist, wobei es ebenfalls möglich ist, dass zwei, genau zwei oder mehr als zwei Elektronikmittel 316 vorgesehen, insbesondere in dem Funktionsbereich 308 vollständig oder teilweise eingebettet sind. Es ist somit denkbar, dass ein Elektronikmittel 316 über die erste Seite bzw. Oberfläche 310 und/oder über die zweite Seite bzw. Oberfläche 312 des Funktionsmittels 306 hinaus ragt oder daran angrenzt. Weiterhin ist denkbar, dass ein Elektronikmittel 316 oder mehrere Elektronikmittel 316 ausschließlich bzw. vollständig zwischen der ersten Seite 310 und der zweiten Seite 312 ausgebildet ist/sind. Bevorzugt handelt es sich bei dem Elektronikmittel 316 um ein Sensormittel, insbesondere ein Temperatursensor und/oder Helligkeitssensor bzw. Lichtsensor und/oder Feuchtigkeitssensor und/oder Bewegungssensor, und/oder ein Akkumulator und/oder eine Prozessoreinrichtung und/oder eine Kommunikationseinrichtung, insbesondere Bluetooth, und/oder ein Display und/oder eine akustische Ausgabeeinrichtung und/oder eine Strahlungsquelle, insbesondere eine Lichtquelle, insbesondere eine LED-Beleuchtung, und/oder ein Einrichtung zur Erzeugung elektrischer Energie, insbesondere eine Solarzelleneinrichtung und/oder ein Piezoelementeinrichtung, insbesondere ein oder mehrere Piezoelemente aufweisend. Bevorzugt ist das Elektronikmittel 316 eine Einrichtung, die mehrere der zuvor genannten Komponenten und/oder Einrichtungen aufweist.

Fig. 11e zeigt ein bevorzugt als thermisch isolierender Verschlussdeckel ausgebildetes Verschlussteilanbringungselements 302. Das Verschlussteilanbringungselement 302 ist hierbei mittels eines bevorzugt als zumindest abschnittsweise und besonders bevorzugt vollständig umlaufenden Positionierelements 304 ausgebildeten Fixierbereichs 308 bevorzugt kraftschlüssig mit dem Kragen 10 eines erfindungsgemäßen Verschlussteils 4 koppelbar.

Fig. 11g zeigt einen Zustand, in dem das in den Figuren 11e und 11f gezeigte Verschlussteilanbringungselement 302 an einem erfindungsgemäßen Verschlussteil 4 angeordnet ist.

Fig. 11h zeigt Beispiele weiterer Verschlussteilanbringungselemente 302, die jeweils mit einem korrespondierend ausgebildeten Kraftübertragungselement 6 eines Verschlussteils 4 zusammenwirken können bzw. koppelbar sind. Solche abhängigen Gestaltungen sind vorteilhaft, da durch das Zusammenwirken des Positionierelements 304 oder der Positionierelemente 304 Momente in das Kraftübertragungselement 6 bzw. die Kraftübertragungselemente 6 des Verschlussteils 4 einleitbar sind. Die in den Fig.11e bis 11g zumindest schematisch dargestellte Kopplungsvariante über den Kragen 10 des Verschlussteils 4 ist ferner vorteilhaft, da sie unabhängig von der jeweiligen Gestaltung des Kraftübertragungselements 6 des Verschlussteils 4 ist.

Fig. 11i zeigt eine Schnittdarstellung eines weiteren bevorzugten Beispiels eines Wärmflaschensicherheitsverschluss-Verschlussteils 4. Gemäß diesem Beispiel weist der Verschlussteil 4 in seinem Zentrum eine sich von der Seite der den Kragen 10 oberseitig begrenzenden Oberfläche 11 in den vom Gewinde 12 umgebenen Bereich hinein erstreckende zentrale Kopplungsstelle 318 aus. Die Kopplungsstelle 318 ist dabei bevorzugt Teil der das Verschlussteil 4 nach obenhin, insbesondere in axialer Richtung, abdichtenden Wandung 319. Bevorzugt bildet die Oberfläche 321 der Kopplungsstelle 318 eine zumindest abschnittsweise konische oder dornförmige Gestalt aus.

Fig. 11k zeigt eine Draufsicht auf das in Fig. 11i gezeigte Verschlussteil 4.

Fig. 11l zeigt eine schematische Unteransicht eines weiteren Beispiels eines erfindungsgemäßen Verschlussteilanbringungselements 302. Das Bezugszeichen 308 kennzeichnet hierbei die erste Seite des Funktionsmittels 306 bzw. eine Unterseite des Funktionsmittels 306 des Verschlussteilanbringungselements 302. Weiterhin ist ersichtlich, dass ein Positioniermittel 304, das bevorzugt korrespondierend mit einer Kopplungsstelle 318 eines Verschlussteils 4 ausgebildet ist, bevorzugt zentral an der Unterseite 310 des Funktionsmittels 306 ausgebildet bzw. angeordnet ist.

Fig. 11m zeigt eine schematische Seitenansicht des in Fig. 11I gezeigten Verschlussteilanbringungselements 302.

Weiterhin gilt für alle Verschlussteilanbringungselemente 302, dass die Form des Funktionsmittels 306 von einer runden, insbesondere scheibenartigen, Form abweichen kann.

Fig. 12a zeigt eine schematische Darstellung einer Draufsicht auf ein zweite Seite 312, insbesondere eine äußere Oberfläche, eines Funktionsmittels 306 eines weiteren bevorzugten Verschlussteilanbringungselements 302. Die zweite Seite 312 des Funktionsmittels 306 bildet hierbei bevorzugt eine dreidimensional gestaltete Kopplungsfläche 314 aus. Bevorzugt dient die Kopplungsfläche 314 zum Koppeln mit einem weiteren Element, insbesondere zum Koppeln mit einem Handbetätigungselement bzw. einem Wärmflaschensicherheitsverschluss-Handbetätigungselement 2. Die Kopplungsfläche 314 bildet dabei bevorzugt eine kreuzartige Form aus. Es ist hierbei jedoch denkbar, dass die Kopplungsoberfläche 314 eine von einer kreuzartigen Form abweichende Form ausbildet.

Fig. 12b zeigt eine Seitenansicht des Verschlussteilanbringungselements 302. Es ist in Zusammenschau mit Fig. 12a ersichtlich, dass die in Fig. 12a sichtbare kreuzförmige Kopplungsfläche 314 als Vertiefung in dem Funktionsmittel 306 bzw. Funktionsbereich 308 ausgebildet ist. Alternativ ist jedoch ebenfalls denkbar, dass die Kopplungsfläche 314 bzw. eine dreidimensionale Kopplungsstruktur 314 als hervorstehend ausgebildet sein kann. An den Funktionsbereich 308 bzw. an das Funktionsmittel 306 schließt sich bevorzugt mindestens ein Positioniermittel 304 an. Bevorzugt ist das Positioniermittel 304 negativ zu einem Kraftübertragungselement 6 des Verschlussteils 4 ausgebildet. Das Positioniermittel 304 ist gemäß dieser Darstellung sternförmig ausgebildet, wobei es alternativ auch andere Formen, insbesondere andere von einem Kreis oder Ring abweichende Formen, ausbilden kann.

Fig. 12c zeigt eine Draufsicht auf die Unterseite 310 des in den Figuren 12a und 12b gezeigten Verschlussteilanbringungselement 302.

Fig. 12d zeigt das durch die Figuren 12a-c bereits dargestellte Verschlussteilanbringungselement 302 in einer perspektivischen Ansicht.

Fig. 12e zeigt rein beispielhaft die ebenfalls in Fig. 10a gezeigte Darstellung eines Verschlussteils 4.

Fig. 12f zeigt eine Schnittdarstellung eines Zusammenbaus des in Fig. 12d gezeigten Verschlussteilanbringungselements 302 und des in Fig. 12e gezeigten Verschlussteils 4.

Bevorzugt kann das bzw. ein entsprechend bzw. analog zu den Figuren 12a-12d gestaltetes Verschlussteilanbringungselement 302 ebenfalls als Zwischenteil oder Adapter bezeichnet werden.

Besonders bevorzugt handelt es sich bei dem Verschlussteilanbringungselement 302 zumindest teilweise um Spritzgussteile oder 3D-Druckteile. Bevorzugt besteht das Verschlussteilanbringungselement 302 zumindest teilweise aus seinem Polymermaterial, insbesondere PE, PA, PLA oder einer Kombination daraus.

Die Figuren 13a-d zeigen verschiedene zumindest keimreduziert, insbesondere steril, verpackte erfindungsgemäße Objekte. Es ist hierbei auch vorstellbar, dass alle denkbaren Kombinationen der in diesen Figuren gezeigten Objekte in einer Verpackung keimreduziert, insbesondere steril, angeordnet sein können. Bevorzugt handelt es sich bei den in diesen Figuren gezeigten Verpackungen 200, um Verpackungen, die volumenmäßig oder massemäßig zumindest teilweise oder mehrheitlich oder vollständig aus einem Polymermaterial, insbesondere aus Polypropylen oder Polyamid oder Polyethylen oder aus Kombinationen aus mindestens zweier dieser Werkstoffe, bestehen bzw. diese aufweisen.

Das Bezugszeichen 202 kennzeichnet einen keimreduzierten, insbesondere sterilen, Bereich, der durch die Verpackung 200 begrenzt wird.

Bei den gezeigten Objekten handelt es sich in Fig. 13a um eine Wärmflasche 14 mit einem mehrteiligen Wärmflaschensicherheitsverschluss 1, in Fig. 13b um ein Verschlussteilanbringungselement 302, in Fig. 13c um ein Wärmflaschensicherheitsverschluss-Verschlussteil 4 und um ein Wärmflaschensicherheitsverschluss-Handbetätigungselement 2, und in Fig. 13d um ein weiteres Verschlussteilanbringungselement 302, insbesondere einen Adapter. Ferner kann bevorzugt ein Wärmflaschenbezug, insbesondere ein textiles Material aufweisend, ebenfalls in einer dieser Verpackungen 200 vorgesehen sein.

Die Verpackung 200 lässt sich bevorzugt nach der Einbringung eines Objekts und anschließender Versiegelung besonders bevorzugt nur infolge einer Zerstörung der Verpackung, d.h. einer Aufhebung der Keimreduzierung bzw. einer Aufhebung eines sterilen Zustands, zur Entnahme des jeweiligen Objekts öffnen.

### Bezugszeichenliste

| | | | |
|---|---|---|---|
| 1 | Mehrteiliger Wärmflaschensicherheitsverschluss | 81 | äußere Kraftübertragungsfläche des zweiten Kraftübertragungselements |
| 2 | Handbetätigungselement oder Wärmflaschensicherheitsverschluss- | 82 | innere Kraftübertragungsfläche des zweiten Kraftübertragungselements |
| | Handbetätigungselement | 90 | Eindringbereich des ersten |
| 3 | Element zur händischen Kontaktierung | | Kraftübertragungselements |
| 4 | Verschlussteil oder Wärmflaschensicherheitsverschluss- Verschlussteil | 92 | umschließende Wandung des ersten Kraftübertragungselements oder umschließende Wandung des ersten |
| 5 | Ausnehmung | | Kraftübertragungselements |
| 6 | erstes Kraftübertragungselement | 94 | Erhebung des ersten Kraftübertragungselements |
| 8 | Zweites Kraftübertragungselement | 96 | verjüngender Anteil |
| 10 | Kragen | 98 | zunehmender Anteil |
| 11 | Kragen in Längsrichtung L begrenzende Oberfläche | 100 | Teilkraftübertragungselement des Verschlussteils |
| 12 | Außengewinde | 101 | Teilkraftübertragungselemente des Handbetätigungselements |
| 13 | Umlaufendes Dichtungselement | | |
| 14 | Wärmflasche | 151 | Innenwandung des Gewindeabschnitts |
| 15 | Gewindestück | 160 | innenliegende Oberfläche des Wandungsanteils 60 |
| 16 | Trichter | | |
| 17 | Innengewinde des Gewindestücks | 161 | innenliegende Oberfläche des Wandungsteils 92 |
| 28 | Außengewinde aufweisende Umfangswandung | 200 | Verpackung |
| 30 | erster Hohlraum bzw. erste Vertiefung | 202 | Aufnahmebereich |
| 31 | zweiter Hohlraum bzw. zweite Vertiefung | 300 | Verschlussteilunterseite |
| | | 302 | Verschlussteilanbringungselement oder Wärmflaschen-sicherheitsverschluss-Verschlussteilanbringungselement |
| 32 | dritter Hohlraum bzw. dritte Vertiefung | | |
| 33 | vierter Hohlraum bzw. vierte Vertiefung | | |
| 34 | Vertiefung fünfter Hohlraum bzw. fünfte | 304 | Positionierelement |
| 35 | zentraler Hohlraum bzw. zentrale | 306 | Funktionsmittel |
| | Vertiefung | 308 | Funktionsbereich |
| 60 | Wandung der Erhebung | 310 | erste Seite des Funktionsmittels |
| 61 | äußere Kraftübertragungsfläche des ersten Kraftübertragungselements bzw. der umschließenden Wandung 92 | 312 | zweite Seite des Funktionsmittels |
| | | 314 | Kopplungsfläche |
| 62 | äußere Kraftübertragungsfläche der Wandung der Erhebung | 316 | Elektronikmittel |
| | | 318 | zentrale Kopplungsstelle |
| 64 | Verbindungswandung | 941 | Zentraler Wandungsanteil der Erhebung |
| 79 | außenliegender Wandungsanteil des zweiten Kraftübertragungselements | | |
| | | A | Abstand |
| 80 | innenliegender Wandungsanteil des zweiten Kraftübertragungselements | L | Längsrichtung |
| | | H1 | Höhe 1 |
| | | H2 | Höhe 2 |

## Patentansprüche

1. Mehrteiliger Wärmflaschensicherheitsverschluss (1),
mindestens umfassend
ein Verschlussteil (4)
mit einem Außengewinde (12) zum Eindrehen in ein Innengewinde einer Wärmflasche,
und
mit einem ersten Kraftübertragungselement (6) zum Übertragen von Momenten zum Einschrauben des Verschlussteils (4) in ein Innengewinde einer Wärmflasche (14) zum wasserdichten Verschließen der Wärmflasche (14) und zum Herausschrauben des Verschlussteils (4) aus einem Innengewinde (17) einer Wärmflasche (14);
und
ein Handbetätigungselement (2)
mit einem zweiten Kraftübertragungselement (8) zum Einleiten der zum Einschrauben und Herausschrauben des Verschlussteils (4) in ein Innengewinde (17) einer Wärmflasche (14) erforderlichen Momente in den Verschlussteil (4),
wobei
das erste Kraftübertragungselement (6) und das zweite Kraftübertragungselement (8) derart korrespondierend ausgebildet sind, dass der Verschlussteil (4) und das Handbetätigungselement (2) unter Bildung einer Wirkverbindung lösbar miteinander koppelbar sind,
wobei das erste Kraftübertragungselement (6) als innenliegendes Kraftübertragungselement ausgeführt ist und durch umgebende Wandungsanteile ausgebildet wird, wobei das zweite Kraftübertragungselement (8) zum zumindest abschnittsweisen Eindringen in das innenliegende Kraftübertragungselement ausgebildet ist,
wobei
zumindest ein Teil eines Eindringbereichs (90) des Verschlussteils (4), in den das zweite Kraftübertragungselement (8) einführbar ist, durch eine den Eindringbereich (90) vollständig umschließende Wandung (92) begrenzt wird,
wobei der Verschlussteil (4) vollständig umlaufend einen Kragen (10) aufweist, wobei der Kragen (10) einen Anstoß zur Begrenzung der maximalen Einschraubtiefe und ein umlaufendes Dichtungselement (13) ausbildet, **dadurch gekennzeichnet dass** das umlaufende Dichtungselement (13)
als eine Erhebung mit einer gebogenen Kontaktoberfläche ausgebildet ist und einstückiger Bestandteil des Kragens (10) und somit des Verschlussteils (2) ist und
wobei der Kragen 10 durch eine Kragenwandung ausgebildet ist, die in Längsrichtung L des Verschlussteils mindestens eine Dicke von 2mm aufweist und wobei sich die Kragenwandung orthogonal zur Längsrichtung L erstreckt,
wobei der Verschlussteil (4) und das Handbetätigungselement (2) Spritzgussteile sind.

2. Mehrteiliger Wärmflaschensicherheitsverschluss nach Anspruch 1,
**dadurch gekennzeichnet, dass**
das Verschlussteil (4) und das Handbetätigungselement (2) derart klemmend Zusammenwirken, dass durch das Zusammenwirken des ersten Kraftübertragungselements (6) und des zweiten Kraftübertragungselements (8) das erste Kraftübertragungselement (6) und das zweite Kraftübertragungselement (8) in einem gekoppelten Zustand gehalten werden.

3. Satz mindestens umfassend
einen mehrteiligen Wärmflaschensicherheitsverschluss gemäß Anspruch 1
und
mindestens eine Wärmflasche (14) mit einer Einfüllöffnung und einem im Bereich der Einfüllöffnung angeordneten oder ausgebildeten Gewinde (17) zum Koppeln mit dem Wärmflaschenverschlussteil (11)
und bevorzugt einem um die Wärmflasche (14) herum anordenbaren Wärmflaschenbezug.

4. Los,
mindestens umfassend
ein von der Verpackung (200) in dem Aufnahmebereich (202) zumindest flüssigkeitsdicht eingeschlossener
Satz gemäß Anspruch 3,
wobei der gesamte von der Verpackung (200) begrenzte Aufnahmebereich (202) keimreduziert, insbesondere steril, ist.

## Claims

1. Multi-part hot-water bottle safety closure (1),
comprising at least
one closure part (4)
having an external screw thread (12) for screwing into an internal screw thread of a hot-water bottle, and
having a first force transmission element (6) for transmitting torque for screwing the closure part (4) into the internal screw thread of a hot-water bottle (14) to water-tight close the hot-water bottle (14) and for unscrewing the closure part (4) out of an internal screw thread (17) of a hot-water bottle (14);
and
a manual actuating element (2)
having a second force transmission element (8) for introducing the torque required to screw and unscrew the closure part (4) into the internal screw thread (17) of a hot-water bottle (14),
wherein
the first force transmission element (6) and the second force transmission element (8) being designed to correspond in such a way that the closure part (4) and the manual actuating element (2) can be releasably coupled to one another by forming an operative connection,
wherein the first force transmission element (6) is designed as an internal force transmission element and is formed by surrounding wall portions, wherein the second force transmission element (8) is designed to penetrate at least in sections into the internal force transmission element,
wherein at least one part of a penetration area (90) of the closure part (4), into which the second force transmission element (8) can be inserted, is delimited by an enclosing wall (92) which completely surrounds the penetration area (90),
wherein the closure part (4) comprises a completely circumferential collar (10), wherein the collar (10) forms an abutment for limiting the maximum screw-in depth and a circumferential sealing element (13),
**characterized in that**
the circumferential sealing element (13) is formed as an elevation with a curved contact surface and is an integral part of the collar (10) and thus of the closure part (2) and
wherein the collar (10) is formed by a collar wall having a thickness of at least 2mm in the longitudinal direction L of the closure part, and wherein the collar wall extends orthogonally to the longitudinal direction L,
wherein the closure part (4) and the manual actuating element (2) are injection molded parts.

2. Multi-part hot-water bottle safety closure according to claim 1,
**characterized in that**
the closure part (4) and the manual actuating element (2) cooperate in such a clampingly manner that due to the cooperation of the first force transmission element (6) and the second force transmission element (8) the first force transmission element (6) and the second force transmission element (8) are kept in a coupled state.

3. Set at least comprising
one multi-part hot-water bottle safety closure (1) according to claim 1,
and
at least one hot-water bottle (14) having a filling opening and a screw thread (17) arranged or formed in the area of the filling opening for coupling with the hot-water bottle closure part (11)
and preferably a hot-water bottle cover arrangeable around the hot-water bottle (14).

4. A lot comprising at least:
a set according to claim 3 enclosed by the packaging (200) in the receiving area (202) in at least a liquid-tight manner,
wherein the entire receiving area (202) delimited by the packaging (200) is germ-reduced, in particular sterile.

## Revendications

1. Fermeture de sécurité pour bouillotte (1) en plusieurs parties, comprenant au moins
une partie de fermeture (4)
avec un filet extérieur (12) pour le vissage dans un filet intérieur d'une bouillotte,
et
avec un premier élément de transmission de force (6) pour la transmission de couples pour le vissage de la partie de fermeture (4) dans un filet intérieur d'une bouillotte (14) pour la fermeture étanche à l'eau de la bouillotte (14) et pour le dévissage de la partie de fermeture (4) d'un filet intérieur (17) d'une bouillotte (14) ; et
un élément d'actionnement manuel (2)
avec un second élément de transmission de force (8) pour l'introduction des couples nécessaires au vissage et au dévissage de la partie de fermeture (4) dans un filet intérieur (17) d'une bouillotte (14) dans la partie de fermeture (4),
dans laquelle
le premier élément de transmission de force (6) et le second élément de transmission de force (8) sont réalisés de façon correspondante de telle manière que la partie de fermeture (4) et l'élément d'actionnement manuel (2) puissent être couplés entre eux de manière détachable en formant une liaison active,
dans laquelle le premier élément de transmission de force (6) est réalisé comme élément de transmission de force intérieur et est réalisé par des parties de paroi environnantes, dans laquelle le second élément de transmission de force (8) est réalisé pour la pénétration au moins par sections dans l'élément de transmission de force intérieur,
dans laquelle
au moins une partie d'une zone de pénétration (90) de la partie de fermeture (4), dans laquelle le second élément de transmission de force (8) peut être introduit, est délimitée par une paroi (92) entourant complètement la zone de pénétration (90),
dans laquelle la partie de fermeture (4) présente de manière complètement périphérique un collet (10), dans laquelle le collet (10) réalise un élément de déclenchement pour la délimitation de la profondeur de vissage maximale et un élément de garniture (13) périphérique, **caractérisée en ce que** l'élément de garniture (13) périphérique est réalisé comme une élévation avec une surface de contact pliée et est un constituant d'un seul tenant du collet (10) et ainsi de la partie de fermeture (2)
et
dans laquelle le collet (10) est réalisé par une paroi de collet qui présente dans le sens longitudinal L de la partie de fermeture au moins une épaisseur de 2 mm et dans laquelle la paroi de collet s'étend orthogonalement au sens longitudinal L,
dans laquelle la partie de fermeture (4) et l'élément d'actionnement manuel (2) sont des parties moulées par injection.

2. Fermeture de sécurité pour bouillotte en plusieurs parties selon la revendication 1,
**caractérisée en ce que**
la partie de fermeture (4) et l'élément d'actionnement manuel (2) coagissent par serrage de telle manière que par la coaction du premier élément de transmission de force (6) et du second élément de transmission de force (8) le premier élément de transmission de force (6) et le second élément de transmission de force (8) soient maintenus dans un état couplé.

3. Ensemble comprenant au moins
une fermeture de sécurité pour bouillotte en plusieurs parties selon la revendication 1,
et
au moins une bouillotte (14) avec une ouverture de remplissage et un filet (17) réalisé ou agencé dans la zone de l'ouverture de remplissage pour le couplage avec la partie de fermeture pour bouillotte (11)
et de préférence un revêtement de bouillotte pouvant être agencé autour de la bouillotte (14).

4. Lot
comprenant au moins
un ensemble enfermé au moins de manière étanche au liquide par l'emballage (200) dans la zone de réception (202) selon la revendication 3,
dans lequel la zone de réception (202) entière délimitée par l'emballage (200) présente une réduction de germes, en particulier est stérile.
